# EUROPEAN PATENT APPLICATION

(11) **EP 4 722 368 A1**
(43) Date of publication of application: **08.04.2026**
(21) Application number: 24815798.4
(22) Date of filing: 24.05.2024
(51) Int. Cl.: C12N 15/113, A61K 31/7088

(54) **NOVEL NUCLEIC ACID CONSTRUCT COMPRISING POLY(A) TAIL HAVING SECONDARY OR TERTIARY STRUCTURE AND USES THEREOF**

(30) Priority: 26.05.2023 KR 20230068195
(71) Applicant: Samsung Biologics Co., Ltd., Incheon 21987 (KR)
(72) Inventor: MOON, Seungtae, Gunpo-si Gyeonggi-do 15862 (KR); CHUNG, Namjin, Seoul 06346 (KR); LEE, Sungyul, Incheon 21997 (KR); PARK, Joori, Suwon-si Gyeonggi-do 16698 (KR); HONG, Hyebeen, Incheon 22008 (KR); LEE, Soyeon, Incheon 21395 (KR); KIM, Hokyeong, Icheon-si Gyeonggi-do 17327 (KR); YANG, Seyoung, Incheon 21387 (KR); KWON, Haenaem, Gwangju-si Gyeonggi-do 12797 (KR); KWON, Oh Sung, Incheon 22021 (KR); EUM, Hyehyeon, Incheon 21982 (KR); YOON, Joon, Incheon 22003 (KR); SHIN, Gwangsu, Incheon 22008 (KR); LEE, Minhyung, Sejong 30153 (KR); KANG, Igojo, Seoul 07039 (KR)
(74) Representative: Mewburn Ellis LLP
(86) International application number: PCT/KR2024/007048
(87) International publication number: WO 2024/248422

(57) **Abstract**

The present invention relates to a novel nucleic acid construct having improved stability and protein expression level and uses thereof and, more specifically, to a nucleic acid construct comprising: a coding region encoding a polypeptide or protein; and a poly(A) tail having a secondary or tertiary structure, and a pharmaceutical composition for vaccine or gene therapy, comprising the nucleic acid construct. A nucleic acid construct platform according to the present invention exhibits more improved stability and protein expression rate in cells without interfering with CDS and UTR sequences and the like, and thus can be generally and effectively used in gene therapy, vaccine fields, and the like.

## Description

### TECHNICAL FIELD

The present invention relates to a novel nucleic acid construct having improved stability and protein expression level and uses thereof and, more specifically, to a nucleic acid construct comprising a coding region encoding a polypeptide or protein, and a poly(A) tail having a secondary or tertiary structure, and a vaccine or a pharmaceutical composition for gene therapy comprising the nucleic acid construct.

### BACKGROUND ART

mRNA preparations produced using *in vitro* transcription (IVT) have recently attracted attention as a potential new drug class for either vaccines or gene therapies. The use of IVT mRNA as a drug is based on delivering genetic messages into the cells of patients to prevent or alter specific disease states (Ugur Sahin, et al., Nat Rev Drug Discov. 2014 Oct;13(10):759-80). Unlike DNA, mRNA produced through IVT for therapeutic applications is not transported to the nucleus but rather acts directly on the cytosol, eliminating concerns about genetic modification, and, similarly to native mRNA, may be engineered to transiently express proteins, reducing concerns about side effects. However, mRNA may be easily degraded by ribonuclease (RNase), which is commonly present both inside and outside cells.

*In vivo,* mRNA degradation is crucial for protein expression and is essential for the effectiveness of therapeutic mRNA preparations (Caroline C Friedel, et al., Nucleic Acids Res. 2009 Sep;37(17):e115). Therefore, various efforts have been made to avoid mRNA degradation and ultimately improve mRNA stability in the process of producing therapeutic mRNAs.

The degradation mechanism of single-stranded mRNA generally involves deadenylation of the poly(A) sequence constituting the 3' end structure by deadenylase, followed by exposure of the 5' end to exonuclease through a decapping mechanism (Nicole L Garneau, et al., Nat Rev Mol Cell Biol. 2007 Feb;8(2):113-26). To protect mRNA from exonucleases, it is known that the 5' end forms a cap structure that binds to cap-binding proteins and the 3' end involves poly(A) binding protein (PABP) that binds to approximately 12-27 repeated adenosine sites, blocking the access of exonucleases (deadenylase) and enhancing the stability thereof (Seung Hwan Lee, et al., Nucleic Acids Res. 2014 Feb;42(4):2697-707). Furthermore, PABP enables mRNA to form a closed loop through protein-protein interactions with translational initiation factors bound to the 5' cap and thus plays a crucial role in enhancing protein translation efficiency.

The poly(A) region, which is commonly referred to as the poly(A) tail, is a simple sequence including repeating adenosine nucleotides. In endogenous environments, 25 to 250 nt adenosine nucleotides are typically synthesized, but the number thereof varies depending on the type of mRNA (Hyeshik Chang, et al., Mol Cell. 2014 Mar 20;53(6):1044-52). Currently, the number of poly(A) nucleotides that may be synthesized *in vitro* is known to be approximately 100. It has been reported that about 80 or more poly(A) nucleotides are randomly cleaved in *E. coli* during pDNA amplification (US 2017/0166905 A). Efforts are continuously being made to enhance mRNA stability and protein expression levels based on stabilization of the poly(A) region.

To date, efforts to enhance the stability and protein expression levels of therapeutic mRNA preparations *in vivo* have focused on optimizing the untranslated region (UTR) and coding sequence (CDS), nucleic acid variants (e.g., m1Ψ), and improving the cap structure. Although the poly(A) tail located at the 3' end of the mRNA structure plays a crucial role in exonuclease-mediated mRNA stability, research associated therewith remains limited.

Regarding the development of mRNA vaccines, US 2021/0128716 A, EP 2831240 B, and US 2018/0185517 A disclose a configuration in which poly(C) and a histone stem-loop structure are inserted into the end of the mRNA poly(A) tail. US 2017/0166905 A discloses a configuration in which an artificial linker-like nucleotide sequence is inserted into the middle of the mRNA poly(A) tail wherein the linker ensures that 100 poly(A) bases are consistently maintained during pDNA amplification and production in *E. coli.*

Each of the technologies used in US 2021/0128716 A and EP 2831240 B demonstrated superiority in protein expression levels compared to the native form when mRNA was produced and delivered into cells. Therefore, these two reports show the importance of optimizing the polyA tail, and ongoing optimization of the polyA region is necessary to increase stability and protein expression.

Under this technical background, as a result of extensive efforts to develop a platform that enhances the initial stability and protein expression rate of intracellular mRNA, and maintains high efficiency and consistency across all mRNA forms without interference with the UTR or CDS, the present inventors identified a platform that can enhance intracellular stability and protein expression levels by forming secondary or tertiary structures, such as stem-loop structures, bulged stem structures, pseudoknot structures, or completely complementary binding structures using a complete complementary binding sequence derived from a virus or gene at the middle and ends of the poly(A) of an mRNA construct that may be used in gene therapies and vaccines. Based thereon, the present invention has been completed.

The information disclosed in this Background section is provided only for better understanding of the background of the present invention, and therefore it may not include information that forms the prior art that is already obvious to those skilled in the art.

### SUMMARY OF THE INVENTION

An object of the present invention is to provide a novel nucleic acid construct with enhanced intracellular stability and protein expression levels.

Another object of the present invention is to provide a vaccine comprising the nucleic acid construct and vaccination using the same.

Still another object of the present invention is to provide a pharmaceutical composition for gene therapy comprising the nucleic acid construct and a gene therapy method using the same.

Yet another object of the present invention is to provide the use of the nucleic acid construct for vaccination and gene therapy, and the use of the nucleic acid construct for the manufacture of a vaccine or a medicament for gene therapy.

In order to accomplish the above objects, the present invention provides a nucleic acid construct comprising a coding region (CDS) encoding a polypeptide or protein; and a poly(A) tail having any one selected from structures represented by Formula (I) to Formula (VIII) .

The present invention also provides a vaccine or a pharmaceutical composition for gene therapy comprising the nucleic acid construct.

The present invention also provides vaccination or gene therapy comprising administering the nucleic acid construct.

The present invention also provides the use of the nucleic acid construct for vaccination or gene therapy.

The present invention also provides the use of the nucleic acid construct for the manufacture of a vaccine or a medicament for gene therapy.

### BRIEF DESCRIPTION OF THE DRAWINGS

FIG. 1 is a schematic diagram illustrating the DENV genome, with three complementary binding sites (5'-3' UAR/DAR/CS) represented by boxes.
FIG. 2 is a schematic diagram illustrating a poly(A) shielding construct using a CS (complementary binding sequence).
FIG. 3 is a schematic diagram illustrating the secondary structure of the poly(A) shielding construct.
FIG. 4 is a schematic diagram illustrating an mRNA expression cassette construct using the DENV UAR/DAR/CS sequence.
FIG. 5 shows the formation of secondary structure through complementary binding using a SHAPE-Map assay which allows structure prediction.
FIG. 6 shows comparison in protein expression rates depending on the presence or absence of the CS sequence.
FIG. 7 is a schematic diagram illustrating mRNA expression cassette constructs according to various combinations of poly(A) numbers.
FIG. 8 is a graph showing the protein expression rates of the constructs shown in FIG. 7.
FIG. 9 is a schematic diagram illustrating mRNA expression cassette constructs comprising, at the CS₂ end, various combinations such as addition of poly(A) (PLA309 to PLA311), a sequence combination referred to as a "mixed tail" or "non-A" (PLA312 to PLA320'), and a sequence combination referred to as an "ARS (adenine rich site)" (such as PLA44).
FIG. 10 is a graph showing the protein expression efficiency of the constructs shown in FIG. 9.
FIG. 11 is a schematic diagram and graph showing the protein expression rates of mRNA expression cassette constructs comprising artificially constructed CS sequences other than the DENV UAR/DAR/CS sequences.
FIG. 12 shows mRNA expression cassette constructs comprising various structures and combinations, such as the DENV CS, artificial sequences, and bulge sequences.
FIG. 13 is a graph showing the protein expression rates of the constructs shown in FIG. 12.
FIG. 14 is a schematic diagram illustrating mRNA expression cassette constructs comprising different CDSs.
FIG. 15 is a graph showing the protein expression rates of the constructs shown in FIG. 14.
FIG. 16 shows mRNA expression cassette constructs comprising different UTRs.
FIG. 17 is a graph showing the protein expression rates of the constructs shown in FIG. 16.
FIG. 18 shows the effect of the mRNA expression cassette construct according to the present invention on the increase of pDNA stability.
FIG. 19 shows the effect of the mRNA expression cassette construct according to the present invention on inhibition of dsRNA production.
FIG. 20 shows the immunogenicity of the mRNA expression cassette construct according to the present invention.

### DETAILED DESCRIPTION OF THE INVENTION AND PREFERRED EMBODIMENTS OF THE INVENTION

Unless defined otherwise, all technical and scientific terms used herein have the same meanings as appreciated by those skilled in the field to which the present invention pertains. In general, the nomenclature used herein is well-known in the art and is ordinarily used.

In one embodiment of the present invention, a platform that can increase the stability and protein expression rate of intracellular mRNA and can always maintain consistently high efficiency in any mRNA sequence without interference with a UTR or CDS sequence was developed by forming a secondary structure or tertiary structure in the form of a stem loop structure, a bulged stem structure, a pseudoknot structure, or a complete complementary binding structure using a complementary binding sequence inside and outside the poly(A) of an mRNA construct.

Accordingly, in one aspect, the present invention is directed to a nucleic acid construct comprising a coding region (CDS) encoding a polypeptide or protein; and a poly(A) tail having any one selected from structures represented by Formula (I) Formula (VIII).

{(A)ₘ-CS₁-(A)ₙ-CS₂-(A)ₒ}ₚ Formula (I)

{ (N)_{q}-(A)ₘ-CS₁-(A)ₙ-CS₂}ₚ Formula (II)

[(A)ₘ-CS₁-{ (A)ᵣ-(G/C/U)}ₓ-(A)_{y}-CS₂]ₚ Formula (III)

[(A)ₘ-CS₁-{(A)ᵣ-(G/C/U)}ₓ-{(A)ₛ-(G/C/U)}_{x'}-CS₂]ₚ Formula (IV)

[{ (A)ᵣ-(G/C/U)}ₓ-(A)_{y}-CS₁-(A)ₙ-CS₂]ₚ Formula (V)

[{(A)ᵣ-(G/C/U)}ₓ-(A)_{y}-CS₁-{(A)ᵣ-(G/C/U)}_{x'}-(A)_{y'}-CS₂]ₚ Formula (VI)

[{(A)ᵣ-(G/C/U)}ₓ-(A) _{y}-CS₁-{(A)ₛ-(G/C/U)}_{x'}-CS₂]ₚ Formula (VII)

[{(A)ᵣ-(G/C/U)}ₓ-(A) _{y}-CS₁-{(A) r- (G/C/U)}x'-{ (A)ₛ-(G/C/U)}_{x'}-CS₂]ₚ Formula (VIII)

In the present invention, G/C/U refers to G (guanine), C (cytosine), or U (uracil). The construct comprising the G/C/U includes a sequence combination designated as a mixed tail or non-A which has a functionally similar function to poly(A).

N refers to a sequence comprising a combination of U (uracil), G (guanine), A (adenine), or C (cytosine), and may be a random base that maintains the structure and function of the poly(A) tail. In one embodiment of the present invention, the N may be a sequence designated as an ARS (adenine rich site) which has a function similar to poly(A).

A may be adenine or a random base that maintains the structure and function of the poly(A) tail.

In Formula (I) to Formula (VIII), m may be an integer of 5 to 200 and n may be an integer of 3 to 200, but is not limited thereto.

In the present invention, at least one of m or n may be an integer of 20 or less, but is not limited thereto.

In the present invention, m may be an integer of 10 to 200, preferably 10 to 150, more preferably 20 to 100, and most preferably 30 to 80, but is not limited thereto.

In the present invention, n may be an integer of 3 to 200, preferably 5 to 100, more preferably 8 to 80, and most preferably 10 to 50, but is not limited thereto.

In Formula (I) to Formula (VIII), o may be an integer from 3 to 200, but is not limited thereto. Preferably, o may be an integer of 12 to 150, more preferably 20 to 100, and most preferably 30 to 70, but is not limited thereto.

In Formula (I) to Formula (VIII), p may be an integer of 1 to 10, q may be an integer of 0 to 200, r and s may be integers of 1 to 30, x and x' may be integers of 1 to 10, and y and y' may be integers of 0 to 80, but is not limited thereto.

In the present invention, the poly A tail may have the structure of Formula (I), m is an integer of 30 to 80, n is an integer of 12 to 50, o is an integer of 12 to 60, and p is an integer of 1 to 5, but is not limited thereto.

In Formula (I) to Formula (VIII), CS₁ and CS₂ may be complementary binding sequences. Any sequence that increases the stability of the nucleic acid construct and protein expression rate without interfering with the UTR or CDS sequence may be applied without limitation.

CS₁ and CS₂ may each independently comprise 5 to 100 nucleotides, preferably 10 to 80, more preferably 20 to 60, and most preferably 30 to 50 nucleotides, but is not limited thereto.

In the present invention, CS₁ and CS₂ may be complementarily bind to each other to form a stem-loop structure, a bulged stem structure, or a pseudoknot structure, or may complementarily bind to each other by 30% to 100%.

CS₁ and CS₂ may complementarily bind to each other by 30% to 100%, 40% to 100%, 50% to 100%, 60% to 100%, 70% to 100%, 80% to 100%, 90% to 100%, 95% to 100%, 96% to 100%, 97% to 100%, 98% to 100%, 99% to 100%, or 100%, but are not limited thereto.

In the present invention, CS₁ may be represented by any one nucleotide sequence of SEQ ID NOs: 1 to 9 and CS₂ may be represented by any one nucleotide sequence of SEQ ID NOs: 10 to 18, but are not limited thereto.
CS₁:
   SEQ ID NO. 1:
      5'-AGAGAGCAGAUCUCUGUUGAAUAACCAACGUCAAUAUGCUG-3'
   SEQ ID NO. 2:
      5'-GAGAGCAGAUCUCUGUUGAAUAACCAACGUCAAUAUGCUG-3'
   SEQ ID NO. 3:
      5'-AUCCGGUUGCGUUGUGAAUCGCGCCACUCCUAACUUGGACC-3'
   SEQ ID NO. 4:
      5'-UCCGGUUGCGUUGUGAAUCGCGCCCACACCUAACUUGGACC-3'
   SEQ ID NO. 5:
      5'-UCCGGUUGCGUUGUGGUUGAAUAACCAACCUAACUUGGACC-3'
   SEQ ID NO. 6:
      5'-CCGUCGAUUGUCCACGUUGAAUAACCAACCGGACGCUAGCC-3'
   SEQ ID NO. 7:
      5'-AGAGCAGAUGUUGAAUAACCAACGUCUG-3'
   SEQ ID NO. 8:
      5'-AGAGAGCAGAUCUGAUACAACGUCAAUGCUG-3'
   SEQ ID NO. 9:
      5'-AGAGAGCAGAUCUCUGUCAAUAUGCUG-3'
CS₂:
   SEQ ID NO. 10:
      5'-CAGCAUAUUGACGCUGGGAAAGACCAGAGAUCUGCUCUCU-3'
   SEQ ID NO. 11:
      5'-GGUCCAAGUUAGGAAAGUAUCAACUCGCAACGCAACCGGA-3'
   SEQ ID NO. 12:
      5'-CAGCAUAUUGACUUGGUUAUUCAACAGAGAUCUGCUCUCU-3'
   SEQ ID NO. 13:
      5'-GGUCCAAGUUAGGCGUGUAUCAACUCGCAACGCAACCGGA-3'
   SEQ ID NO. 14:
      5'-GGUCCAAGUUAGGCUGGGAAAGACCCGCAACGCAACCGGA-3'
   SEQ ID NO. 15:
      5'-GGCUAGCGUCCGGCUGGGAAAGACCGUGGACAAUCGACGG-3'
   SEQ ID NO. 16:
      5'-CAGACGCUGGGAAAGACCAUCUGCUCU-3'
   SEQ ID NO. 17:
      5'-CAGCAUUGACGCUGGAGAGAUCUGCUCUCU-3'
   SEQ ID NO. 18:
      5'-CAGCAUAUUGACAGAGAUCUGCUCUCU-3'

Preferably, CS₁ and CS₂ may have sequences that do not cause pathogenic effects derived from RNA viruses. More preferably, CS₁ and CS₂ may have conserved sequences derived from dengue virus (DENV), but are not limited thereto.

In the present invention, the poly A tail may have any one of nucleotide sequences represented by SEQ ID NO: 24, and SEQ ID NOs: 29 to 102, but is not limited thereto.

As used herein, the term "nucleic acid" preferably refers to DNA or RNA. In the present invention, "nucleic acid construct" may be used to encompass a DNA construct, an RNA construct, an mRNA construct, or the like, and may preferably be an mRNA construct, but is not limited thereto.

In relation to the nucleic acid, the terms "polynucleotide", "nucleotide", "nucleotide sequence" and "oligonucleotide" are used interchangeably. Polynucleotides of any length may include polymeric forms of nucleotides, deoxyribonucleotides, ribonucleotides, or analogues thereof. Polynucleotides may have any three-dimensional structure and may perform any known or unknown functions. Polynucleotides may include one or more modified nucleotides, such as methylated nucleotides and nucleotide analogues. Modifications to the nucleotide structure may be possible before or after polymer assembly.

Preferably, a nucleic acid is a polymer comprising or consisting of nucleotide monomers covalently linked to each other by phosphodiester bonds in a sugar/phosphate backbone. "Nucleic acid" may include modified nucleic acids such as DNA or RNA molecules with base modifications, sugar modifications, or backbone modifications.

In the present invention, the nucleic acid may include an unmodified or modified nucleic acid. The modified nucleic acid may be selected from the group consisting of pseudouridine (Ψ), N1-methylpseudouridine (m1Ψ), 5-methyluridine (m5U), 2-thiouridine (s2U), 2'-O-methyluridine (2'-O-methyl-U, Um), 5-methylcytidine (m5C), and 5-methoxyuridine (5moU), but is not limited thereto.

As used herein, the term "DNA" is an abbreviation for deoxyribonucleic acid and is a polymer composed of nucleic acid molecules or nucleotides. These nucleotides are typically deoxy-adenosine-monophosphate, deoxythymidine-monophosphate, deoxy-guanosine-monophosphate, and deoxy-cytidine monophosphate monomers. The nucleotides consist of a sugar (deoxyribose), a base, and a phosphate, and are polymerized by a unique backbone structure. The backbone structure is typically formed by the sugar moiety of the first nucleotide, that is, deoxyribose, the phosphate moiety of the second nucleotide, and phosphodiester bonds between adjacent monomers. The specific order of these monomers, i.e., the order of the bases linked to the sugar/phosphate backbone, is called a DNA sequence. DNA may be single-stranded or double-stranded. In double-stranded form, nucleotides in the first strand typically hybridize with nucleotides in the second strand, for example, through A/T base pairing and G/C base pairing.

As used herein, the term "RNA" is an abbreviation for ribonucleic acid, a nucleic acid molecule, i.e., a polymer composed of nucleotides. For example, RNA includes messenger RNA (mRNA). Nucleotides are typically adenosine-monophosphate, uridine-monophosphate, guanosine-monophosphate, and cytidine monophosphate monomers linked together via a so-called backbone. The backbone is formed primarily by a sugar, such as ribose, and by phosphodiester bonds between adjacent monomers, the phosphate moieties. The specific sequence of monomers is called an RNA sequence. Messenger RNA typically provides a nucleotide sequence that may be translated into the amino acid sequence of a specific peptide or protein. Typically, mRNA includes a 5'-cap, a 5'-UTR, an open reading frame (ORF) (or CDS), a 3'-UTR, and a poly(A) sequence. Aside from messenger RNA, there are several non-coding forms of RNA that may be involved in the regulation of transcription and/or translation.

In the present invention, the nucleic acid construct may further comprise a 5'-untranslated region (5'-UTR) and a 3'-untranslated region (3'-UTR) bound to opposite ends of the coding region; and a 5'-cap or IRES bound to the 5'-untranslated region, but is not limited thereto. Preferably, the nucleic acid construct may have a structure represented by the following Formula (IX) or Formula (X), but is not limited thereto.

Formula (IX): 5'-cap - 5'-UTR - CDS - 3'-UTR-[Formula (I) to Formula (VIII)]

Formula (X): 5' - IRES - 5'-UTR - CDS - 3'-UTR-[Formula (I) to Formula (VIII)]

As used herein, the term "5'-untranslated region (5'-UTR)" refers to a nucleic acid molecule that is located 5' (i.e., "upstream") of a coding region and is not translated into a protein. Typically, the 5'-UTR begins at the transcription start site and ends one nucleotide before the start codon of the coding region. Preferably, the 5'-UTR is 20, 30, 40, or 50 or more nucleotides in length. The 5'-UTR may contain elements for regulating gene expression, so-called regulatory elements. Such regulatory elements may be, for example, ribosome binding sites. The 5'-UTR may be post-transcriptionally modified, for example, by the addition of a 5'-cap. The 5'-UTR of an mRNA is not translated into an amino acid sequence. The 5'-UTR sequence is typically encoded by a gene, which is transcribed into each mRNA during gene expression. The genomic sequence is first transcribed into a premature mRNA containing an optional intron. The premature mRNA is further processed into a mature mRNA during a maturation process. This maturation process includes 5'-capping, splicing of the premature mRNA to truncate the premature mRNA into optional introns, and modification of the 3'-end, such as polyadenylation of the 3'-end of the premature mRNA. In the present invention, the 5'-UTR corresponds to the sequence of the mature mRNA located between the start codon and, for example, the 5'-cap. Preferably, the 5'-UTR corresponds to a sequence extending from a nucleotide located 3' to the 5'-cap, more preferably a nucleotide immediately 3' to the 5'-cap, to a nucleotide located 5' to the start codon of the protein coding region, preferably a nucleotide immediately 5' to the start codon of the protein coding region. The nucleotide immediately 3' to the 5'-cap of the mature mRNA typically corresponds to the transcription start site. The term "corresponding" means that the 5'-UTR sequence may be an RNA sequence, such as the mRNA sequence used to define the 5'-UTR sequence, or a DNA sequence corresponding to such an RNA sequence.

As used herein, the term "3'-untranslated region (3'-UTR)" refers to a portion of an artificial nucleic acid molecule located 3' (i.e., "downstream") of the coding region and not translated into protein. Typically, the 3'-UTR is the region of an mRNA located between the protein coding region (ORF or CDS) and the poly(A) sequence of the mRNA. As used herein, the term 3'-UTR may include an element not encoded by the template from which the RNA is transcribed. The 3'-UTR sequence is typically encoded by a gene that is transcribed into mRNA during gene expression. In the present invention, the 3'-UTR corresponds to a sequence of a mature mRNA located between the stop codons of the protein coding region, preferably immediately 3' to the stop codon of the protein coding region. The term "corresponding" means that the 3'-UTR sequence may be an RNA sequence, such as the mRNA sequence used to define the 3'-UTR sequence, or a DNA sequence corresponding to such an RNA sequence. Preferably, the 3'-UTR is at least 20, 30, 40, or 50 nucleotides in length.

In the present invention, the term "5'-cap" refers to an independent component that "caps" the 5' end located at the 5' start site of an mRNA. Typically, the cap structure serves to initiate protein synthesis and protect the mRNA from the action of nucleases. The 5'-cap may typically be formed by modified nucleotides, particularly derivatives of guanine nucleotides. Preferably, the 5'-cap is linked to the 5' end via a 5'-5'-triphosphate linkage. The 5'-cap is in a methylated form, for example, m7GpppN, wherein N is the end 5' nucleotide of the nucleic acid linked to the 5'-cap, typically the 5'-end of RNA.

Additional examples of 5'-cap structures include glyceryl, inverted deoxy abasic residue (partially), 4',5' methylene nucleotides, 1-(beta-D-erythrofuranosyl) nucleotides, 4'-thio nucleotides, carbocyclic nucleotides, 1,5-anhydrohexitol nucleotides, L-nucleotides, alpha-nucleotides, modified base nucleotides, threo-pentofuranosyl nucleotides, acyclic 3',4'-seconucleotides, acyclic 3,4-dihydroxybutyl nucleotides, acyclic 3,5 dihydroxypentyl nucleotides, 3'-3'-inverted nucleotide moieties, 3'-3'-inverted abasic residue moieties, 3'-2'-inverted nucleotide moieties, 3'-2'-inverted abasic moieties, 1,4-butanediol phosphate, 3'-phosphoramidate, hexyl phosphate, aminohexyl phosphate, 3'-phosphate, 3'-phosphorothioate, phosphorodithioate, or a bridging or non-bridging methylphosphonate moiety.

As used herein, the term "IRES (internal ribosome entry site)" refers to an internal ribosome entry point or ribosome binding site. It is known to form a loop structure on mRNA and induce translation initiation through a cap-independent mechanism. When a single vector is produced by positioning an IRES between two or more genes using IRES, two genes may be simultaneously expressed on a single mRNA. Therefore, inserting an IRES region to induce target gene expression is widely used in genetic recombination technology.

As used herein, the term "CDS (coding sequence)" refers to a coding region encoding a target polypeptide or protein, and is used interchangeably with ORF (open reading frame).

The polypeptide or protein may be, but is not limited to: (a) a therapeutically active protein or peptide; or (b) an antigen selected from the group consisting of tumor antigens, pathogenic antigens, viral antigens, protozoal antigens, bacterial antigens, allergens, and autoimmune antigens. Depending on the gene to be inserted into the coding region, the nucleic acid construct according to the present invention may be used for gene vaccine therapy or gene therapy for autoimmune diseases, infectious diseases, cancer or tumor-related diseases, inflammatory diseases, and the like.

Specifically, when the nucleic acid construct according to the present invention is used as a vaccine for vaccine therapy, the CDS may be a region encoding all or part of a region of a viral antigen that may cause infection in humans and animals, such as influenza virus, coronavirus, varicella zoster virus, human papillomavirus, Zika virus, herpes virus, AIDS virus, SFTS virus, measles virus, Ebola virus, MERS virus, hepatitis virus, avian influenza, rabies virus, foot-and-mouth disease virus, respiratory syncytial virus, or the like, but is not limited thereto.

In addition, when the nucleic acid construct of the present invention is used as a gene therapy agent by expressing a protein or antigen, the CDS may be a region encoding all or part of the sequence of a protein drug, antibody, disease-related antigen, or the like, but is not limited thereto.

The disease-related antigens may be selected from the group consisting of 4-1BB, integrin, amyloid beta, angiopoietin (angiopoietin 1 or 2), angiopoietin-like substance 3, B-cell activating factor (BAFF), BAFF-R, BCMA, B7-H3, complement 5, CCR4, CD3, CD4, CD6, CD11a, CD19, CD20, CD22, CD30, CD33, CD38, CD52, CD62, CD79b, CD80, CGRP, claudin-18, complement factor D, CTLA4, DLL3, EGF receptor, hemophilia factor, FGF23, folate receptor, GD2, GM-CSF, HER2, HER3, interferon receptor, interferon gamma, IgE, IGF-1 receptor, interleukin 1, interleukin 2, interleukin 2 receptor, interleukin 4, interleukin 4 receptor, interleukin 5, interleukin 5 receptor, interleukin 6, interleukin 6 receptor, interleukin 7, interleukin 12/23, interleukin 13, interleukin 17A, interleukin 17 receptor A, interleukin 31 receptor, interleukin 36 receptor, TGF-beta, TGF-beta receptor, LAG3, LFA3, NGF, PVSK9, PD-1, PD-L1, TIGIT, TIM3, GITR, KLRG1, OX40, OX40L, RANK-L, SLAMF7, tissue factor, TNF, VEGF, and VEGF receptor, but is not limited thereto.

The antibody may be used for the treatment of conditions associated with a disease, preferably a therapeutic antibody targeting an antigen associated with the disease, and more preferably an antibody drug currently on the market or in the clinical trial phase, but is not limited thereto.

Furthermore, the protein drug refers to a drug composed of proteins, including antibody drugs, that is composed of amino acids and exhibits a disease-treating or preventive effect through protein activity. The protein drug may be selected from the group consisting of cytokines, therapeutic enzymes, hormones, soluble receptors and fusion proteins thereof, insulin or analogs thereof, BMP (bone morphogenetic protein), EPO (erythropoietin), and serum-derived proteins, but is not limited thereto.

As a specific example, the cytokine may be selected from the group consisting of interferon, interleukin, colony stimulating factor (CSF), tumor necrosis factor (TNF), and tissue growth factor (TGF), but is not limited thereto, and the therapeutic enzyme may be selected from the group consisting of beta-glucocerebrosidase and agalsidase beta, but is not limited thereto.

In addition, the soluble receptor refers to an extracellular domain of the receptor and the fusion protein thereof refers to a protein in which the soluble receptor is fused with the Fc region of an antibody or the like, and includes a configuration in which the Fc region is fused to a TNF-α soluble receptor (e.g., a product with the generic name Etanercept), a configuration in which the Fc region is fused to a VEGF soluble receptor (e.g., a product with the generic name "Aflibercept" and similar forms), a configuration in which the Fc region is fused to CTLA-4 (e.g., a product with the generic name Abatacept or Belatacept and similar forms), a configuration in which the Fc region is fused to an interleukin-1 soluble receptor (e.g., a product with the generic name Rilonacept and similar forms), and a configuration in which the Fc region is fused to the LFA3 soluble receptor (e.g., a product with the generic name Alefacept and similar forms) may be exemplified, but are not limited thereto.

The hormone refers to a hormone or an analogue thereof injected externally for the treatment or prevention of diseases caused by hormone deficiency, or the like and examples thereof include, but are not limited to, human growth hormone, estrogen, and progesterone. The plasma-derived protein refers to a protein present in plasma and refers to both proteins extracted from plasma and proteins produced recombinantly. Examples of the plasma-derived protein include fibrinogen, von Willebrand factor, albumin, thrombin, FII (factor II), FV (factor V), FVII (factor VII), FVIII (factor VIII), FIX (factor IX), FX (factor X), and FXI (factor XI), but are not limited thereto.

In the present invention, template DNA (e.g., plasmid DNA (pDNA)) may be produced to establish a nucleic acid construct comprising mRNA of a gene encoding a target polypeptide or protein using *in vitro* transcription (IVT).

The mRNA produced using the IVT may be purified using a purification method known in the art. Examples of purification methods include, but are not limited to, multimodal chromatography, reversed-phase high-performance liquid chromatography (RP-HPLC), size-exclusion HPLC (SEC), ion exchange HPLC (IEC), cellulose chromatography, oligo-deoxythymidine acid (oligo-dT), or tangential flow filtration (TFF).

In the present invention, the plasmid DNA for expressing the nucleic acid construct using the IVT method may further include a promoter sequence. The promoter may be located upstream of the 5'-UTR. The promoter may include an element necessary for transcription, such as, for example, an RNA polymerase promoter. The promoter may include a phage RNA polymerase promoter such as T7, SP6 or T3, preferably a T7 or SP6 promoter encoding an mRNA sequence.

In another aspect, the present invention is directed to a vaccine or a pharmaceutical composition for gene therapy comprising the nucleic acid construct.

In still another aspect, the present invention is directed to vaccination or gene therapy comprising administering the nucleic acid construct to a subject in need of treatment or prevention.

In yet another aspect, the present invention is directed to the use of the nucleic acid construct for vaccination or gene therapy.

In still yet another aspect, the present invention is directed to the use of the nucleic acid construct for the manufacture of a vaccine or a medicament for gene therapy.

As used herein, the term "vaccine" refers to a prophylactic or therapeutic substance that provides at least one antigen, preferably an immunogen. The antigen or immunogen may be derived from any material suitable for vaccination. For example, the antigen or immunogen may be derived from a pathogen, such as a bacterial or viral particle, or from a tumor or cancerous tissue. The antigen or immunogen stimulates the body's adaptive immune system, which provides an adaptive immune response.

As used herein, the term "prevention" refers to any action that prevents the onset of a disease or delays its progression by administration of the composition. Furthermore, as used herein, the term "treatment" refers to any action that ameliorates, alleviates, or completely cures the symptoms of a disease by administration of the composition.

As used herein, the term "medicament for gene therapy" may include an anticancer agent, an immunotherapy agent, a CAR-T therapeutic agent, or a therapeutic agent using CRISPR and derivatives thereof, but is not limited thereto. The description of the nucleic acid construct of the present invention used as a medicament for gene therapy may also apply thereto.

In the present invention, the pharmaceutical composition may comprise a delivery vehicle for delivering mRNA.

The nucleic acid construct according to the present invention may be delivered via liposomes, LNPs (lipid nano-particles), or various nanoparticles.

Liposomes or LNPs contain cationic lipids, non-cationic lipids, neutral lipids or the like, and may further contain other lipids such as polyethylene glycol (PEG) or cholesterol. These mRNA transports are specifically disclosed in US 2018/0311176 A, US 2019/0032051 A, US 2021/0046192 A, WO 2018/081480 A, WO 2020/097540 A, WO 2020/097548 A, and WO 2021/007278 A, which are incorporated herein by reference.

The cationic lipids are specifically exemplified by US 2018/0311176 A, US 2019/0032051 A, and the like, and examples thereof include N,N-dioleyl-N,N-dimethylammonium chloride (DODAC), N,N-distearyl-N,N-dimethylammonium bromide (DDAB), N-(1-(2,3-dioleyloxy)propyl)-N,N,N-trimethylammonium chloride (DOTAP), N-(1-(2,3-dioleyloxy)propyl)-N,N,N-trimethylammonium chloride (DOTMA), N,N-dimethyl-2,3-dioleyloxy)propylamine (DODMA), 1,2-dilinoleyloxy-N,N-dimethylaminopropane (DLinDMA), 1,2-dilinolenyloxy-N,N-dimethylaminopropane (DLenDMA), 1,2-dilinoleylcarbamoyloxy-3-dimethylaminopropane (DLin-C-DAP), 1,2-dilinoleyoxy-3-(dimethylamino)acetoxypropane (DLin-DAC), 1,2-dilinoleyoxy-3-morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleyloxy-3-(N-methylpiperazino)propane (DLin-MPZ), 3-(N,N-dilinoleylamino)-1,2-propanediol (DLinAP), 3-(N,N-dioleylamino)-1,2-propanedio (DOAP), 1,2-dilinoleyloxo-3-(2-N,N-dimethylamino)ethoxypropane (DLin-EG-DMA), 1,2-dilinolenyloxy-N,N-dimethylaminopropane (DLinDMA), 2,2-dilinoleyl-4-dimethylaminomethyl-[1,3]-dioxolane (DLin-K-DMA) or analogues thereof, (3aR,5s,6aS)-N,N-dimethyl-2,2-di((9Z,12Z)-octadeca-9, 12-dienyl)tetrahydro-3aH-cyclopenta[d][1,3]dioxol-5-amine (ALN100), (6Z,9Z,28Z,31Z)-heptatriaconta-6,9,28,31-tetraen-19-yl 4-(dimethylamino)butanoate (MC3), 1,1'-(2-(4-(2-((2-(bis(2-hydroxydodecyl)amino)ethyl)(2-hydroxydodecyl)amino)ethyl)piperazin-1-yl)ethylazanediyl)didodecan-2-ol (Tech G1), 2,2-dilinoleyl-4-dimethylaminoethyl-[1,3]-dioxolane, β-L-arginyl-2, 3-L-diaminopropionic acid-N-palmityl-N-oleylamide trihydrochloride, N',N'-dioctadecyl-N-4, 8-diaza-10-aminodecanoylglycine amide [71], 1,2-dilinoleyloxy-3-dimethylaminopropane, DLin-KC2-DMA, amino lipid 2,2-dilinoleyl-4-dimethylaminoethyl-[1,3]-dioxolane (DLin-KC2-DMA, 1), 1,2-distearloxy-N,N-dimethylaminopropane (DSDMA), dilinoleylmethyl-4-dimethylaminobutyrate (DLin-MC3-DMA), DLin-D-DMA, C12-200, 98N12-5, (20Z,23Z)-N,N-dimethylnonacosa-20,23-dien-10-amine, (17Z,20Z)-N,N-dimemylhexacosa-17,20-dien-9-amine, (1Z,19Z)-N5N-dimethylpentacosa-16,19-dien-8-amine, (13Z,16Z)-N,N-dimethyldocosa-13,16-dien-5-amine, (12Z,15Z)-N,N-dimethylhenicosa-12,15-dien-4-amine, (14Z,17Z)-N,N-dimethyltricosa-14,17-dien-6-amine, (15Z,18Z)-N,N-dimethyltetracosa-15,18-dien-7-amine, (18Z,21Z)-N,N-dimethylheptacosa-18,21-dien-10-amine, (15Z,18Z)-N,N-dimethyltetracosa-15,18-dien-5-amine, (14Z,17Z)-N,N-dimethyltricosa-14,17-dien-4-amine, (19Z,22Z)-N,N-dimethyloctacosa-19,22-dien-9-amine, (18Z,21Z)-N,N-dimethylheptacosa-18,21-dien-8-amine, (17Z,20Z)-N,N-dimethylhexacosa-17,20-dien-7-amine, (16Z,19Z)-N,N-dimethylpentacosa-16,19-dien-6-amine, (22Z,25Z)-N,N-dimethylhentriaconta-22,25-dien-10-amine, (21Z,24Z)-N,N-dimethyltriaconta-21,24-dien-9-amine, (18Z)-N,N-dimethylheptacos-18-en-10-amine, (17Z)-N,N-dimethylhexacos-17-en-9-amine, (19Z,22Z)-N,N-dimethyloctacosa-19,22-dien-7-amine, N,N-dimethylheptacosan-10-amine, (20Z,23Z)-N-ethyl-N-methylnonacosa-20,23-dien-10-amine, 1-[(11Z,14Z)-1-nonylicosa-11,14-dien-1-yl]pyrrolidine, (20Z)-N,N-dimethylheptacos-20-en-10-amine, (15Z)-N,N-dimethyleptacos-15-en-10-amine, (14Z)-N,N-dimethylnonacos-14-en-10-amine, (17Z)-N,N-dimethylnonacos-17-en-10-amine, (24Z)-N,N-dimethyltritriacont-24-en-10-amine, (20Z)-N,N-dimethylnonacos-20-en-10-amine, (22Z)-N,N-dimethylhentriacont-22-en-10-amine, (16Z)-N,N-dimethylpentacos-16-en-8-amine, (12Z,15Z)-N,N-dimethyl-2-nonylhenicosa-12,15-dien-1-amine, (13Z,16Z)-N,N-dimethyl-3-nonyldocosa-13,16-dien-1-amine, N,N-dimethyl-1-[(1S,2R)-2-octylcyclopropyl]eptadecan-8-amine, 1-[(1S,2R)-2-hexylcyclopropyl]-N,N-dimethylnonadecan-10-amine, N,N-dimethyl-1-[(1S,2R)-2-octylcyclopropyl]nonadecan-10-amine, N,N-dimethyl-21-[(1S,2R)-2-octylcyclopropyl]henicosan-10-amine,N,N-dimeth-yl-1-[(1S,2S)-2-{[(1R,2R)-2-pentylcyclopropyl]methyl}cyclopropyl]nonadecan-10-amine, N,N-dimethyl-1-[(1S,2R)-2-octylcyclopropyl]hexadecan-8-amine, N,N-dimethyl-[(1R,2S)-2-undecylcyclopropyl]tetradecan-5-amine, N,N-dimethyl-3-{7-[(1S,2R)-2-octylcyclopropyl]heptyl}dodecan-1-amine, 1-[(1R,2S)-2-heptylcyclopropyl]-N,N-dimethyloctadecan-9-amine, 1-[(1S,2R)-2-decylcyclopropyl]-N,N-dimethylpentadecan-6-amine, N,N-dimethyl-1-[(1S,2R)-2-octylcyclopropyl]pentadecan-8-amine, R-N,N-dimethyl-1-[(9Z,12Z)-octadeca-9,12-dien-1-yloxy]-3-(octyloxy)propa-n-2-amine, S-N,N-dimethyl-1-[(9Z,12Z)-octadeca-9,12-dien-1-yloxy]-3-(octy-loxy)propan-2-amine, 1-{2-[(9Z,12Z)-octadeca-9,12-dien-1-yloxy]-1-Roctyloxy)methyl]ethyl}pyrro-lidine, (2S)-N,N-dimethyl-1-[(9Z,12Z)-octadeca-9,12-dien-1-yloxy]-3-[(5Z)-oct-5-en-1-yloxy]propan-2-amine, 1-{2-[(9Z,12Z)-octadeca-9,12-dien-1-yloxy]-1-[(octyloxy)methyl]ethyl}azet-idine, (2S)-1-(hexyloxy)-N,N-dimethyl-3-[(9Z,12Z)-octadeca-9,12-dienylo-xy]propan-2-amine, (2S)-1-(heptyloxy)-N,N-dimethyl-3-[(9Z,12Z)-octadeca-9,12-dien-1-yloxy]pr-opan-2-amine, N,N-dimethyl-1-(nonyloxy)-3-[(9Z,12Z)-octadeca-9,12-dien-1-yloxy]propan-2-amine, N,N-dimethyl-1-[(9Z)-octadec-9-en-1-yloxy]-3-(octyloxy)propan-2-am-ine; (2S)-N,N-dimethyl-1-[(62,92,12Z)-octadeca-6,9,12-trien-1-yloxy]-3-(o-ctyloxy)propan-2-amine, (2 S)-1-[(11Z,14Z)-icosa-11,14-dien-1-yloxy]-N,N-dimethyl-3-(pentyloxy)propa-n-2-amine, (2S)-1-(hexyloxy)-3-[(11Z,14Z)-icosa-11,14-dien-1-yloxy]-N,N-di-methylpropan-2-amine, 1-[(11Z,14Z)-icosa-11,14-dien-1-yloxy]-N,N-dimethyl-3-(octyloxy)propan-2-amine, 1-[(13Z,16Z)-docosa-13,16-dien-1-yloxy]-N,N-dimethyl-3-(octyloxy)pr-opan-2-amine, (2S)-1-[(13Z,16Z)-docosa-13,16-dien-1-yloxy]-3-(hexyloxy)-N,N-dimethylpropan-2-amine, (2S)-1-[(13Z)-docos-13-en-1-yloxy]-3-(hexyloxy)-N,N-dimethylpropan-2-amin-e, 1-[(13Z)-docos-13-en-1-yloxy]-N,N-dimethyl-3-(octyloxy)propan-2-amine, 1-[(9Z)-hexadec-9-en-1-yloxy]-N,N-dimethyl-3-(octyloxy)propan-2-amine, (2R)-N,N-dimethyl-H(1-metoyloctyl)oxy]-3-[(9Z,12Z)-octadeca-9,12-dien-1-yloxy]propan-2-amine, (2R)-1-[(3,7-dimethyloctyl)oxy]-N,N-dimethyl-3-[(9Z,12Z)-octadeca-9,12-di-en-1-yloxy]propan-2-amine, N,N-dimethyl-1-(octyloxy)-3-({8-[(1S,2S)-2-{[(1R,2R)-2-pentylcyclopropyl]-methyl}cyclopropyl]octyl}oxy)propan-2-amine, N,N-dimethyl-1-{[8-(2-oclylcyclopropyl)octyl]oxy}-3-(octyloxy)propan-2-am-ine and (11E,20Z,23Z)-N,N-dimethylnonacosa-11,20,2-trien-10-amine, 5-carboxyspermylglycine dioctaoleoylamide ("DOGS"), dipalmitoylphosphatidylethanolamine 5-carboxyspermyl-amide ("DPPES"), 1,2-dimyristyloxypropyl-3-dimethylhydroxy ethyl ammonium bromide (DMRIE), DMRIE-HP, Lipofectamine (DOSPA), 3b-(N-(N',N'-dimethylaminoethane)-carbamoyl)cholesterol ("DC-Choi"), N-(1,2-dimyhstyloxyprop-3-yl)-N,N-dimethyl-N-hydroxyethyl ammonium bromide ("DMRIE"), 1,2-Dioleoyl-3-dimethylammonium-propane ("DODAP"), DMDMA, cationic lipid-based transfection reagents TransIT-TKO, LIPOFECTIN, Lipofectamine, OLIGOFECTAMINE or DHARMAFECT, DSDMA, DODMA, DLinDMA, DLenDMA, gamma-DLenDMA, DLin-K-DMA, DLin-K-C2-DMA (also known as DLin-C2K-DMA, XTC2, and C2K), DLin-K-C3-DM A, DLin-K-C4-DMA, DLen-C2K-DMA, y-DLen-C2K-DMA, DLin-M-C2-DMA (also known as MC2), DLin-M-C3-DMA (also known as MC3) or (DLin-MP-DMA) (also known as 1-B11), or mixtures thereof, but is not limited thereto.

The non-cationic lipids are specifically exemplified in US 2018/0311176 A, US 2019/0032051 A, and the like and, for example, the non-cationic lipid includes phosphatidylglycerol, cardiolipin, diacylphosphatidylserine, diacylphosphatidic acid, N-dodecanoyl phosphatidylethanoloamine, N-succinyl phosphatidylethanolamine, N-glutaryl phosphatidylethanolamine, or lysylphosphatidylglycerol. In some cases, the non-cationic lipid may be, for example, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), palmitoyloleoylphosphatidylcholine (POPC), palmitoyloleoylphosphatidylethanolamine (POPE), dioleoylphosphatidylethanolamine 4-(-maleimidomethyl)-cyclohexane-1-carboxylate (DOPE-mal), dipalmitoyl phosphatidyl ethanolamine (DPPE), dimyristoylphosphoethanolamine (DMPE), distearoylphosphatidyl-ethanolamine (DSPE), 16-O-monomethyl PE, 16-O-dimethyl PE, 18-1-trans PE, 1-stearoyl-2-oleoylphosphatidyethanolamine (SOPE), cholesterol, phosphatidylglycerols, cardiolipins, diacylphosphatidylserines, diacylphosphatidic acids, N-dodecanoyl phosphatidylethanolamines, N-succinyl phosphatidylethanolamines, N-glutarylphosphatidylethanolamines, lysylphosphatidylglycerols, palmitoyloleyolphosphatidylglycerol (POPG), but is not limited thereto.

The neutral lipids are specifically exemplified in US 2018/0311176 A, US 2019/0032051 A, and the like and, neutral lipids may, for example, include diacylphosphatidylcholine, diacylphosphatidylethanolamine, ceramide, sphingomyelin, dihydrosphingomyelin, cephalin, or cerebrosides.

To prevent aggregation of particles produced during the mRNA delivery, the composition may comprise a PEG fat. The PEG fat is specifically exemplified in US 2018/0311176 A, US 2019/0032051 A, or the like, and may, for example, include PEG-diacylglycerol (DAG), PEG-dialkyloxypropyl (DAA), PEG-phospholipid, PEG-ceramide (Cer), or mixtures thereof, but is not limited thereto.

A further aspect, the nucleic acid construct according to the present invention may be delivered via nanoparticles, for example, gold nanoparticles. The gold nanoparticles may have surface modifications. Specific examples of such modifications are disclosed in Acc Chem Res. 2019 June 18; 52(6): 1496-1506 and Pharmaceutics 2021, 13, 900, which are incorporated herein by reference.

The nucleic acid construct may be linked to gold nanoparticles and form a complex with a cationic endosomal disruptive polymer, which may be delivered into cells (Nature Biomedical Engineering volume 1, pages 889-901 (2017)). The cationic endosomal disruptive polymer may be, for example, a block copolymer of polyethylene glycol (PEG), poly(arginine), poly(lysine), poly(histidine), poly-[2-{(2-aminoethyl)amino}-ethylaspartamide] (pAsp(DET)), poly(ethylene glycol) (PEG) and poly(arginine), a block copolymer of PEG and poly(lysine), or a block copolymer of PEG and poly{N-[N-(2-aminoethyl)-2-aminoethyl]aspartamide} (PEG-pAsp(DET)).

In some cases, gold particles having surfaces modified with arginine may be used. The arginine-modified gold particles may be assembled with a nuclease or a polynucleotide encoding the same, and/or a cleavage factor or a polynucleotide encoding the same, thereby enabling fusion with the membrane of the target cell and movement into the cytoplasm (ACS Nano. 2017, 11:2452-2458).

Still a further aspect, a peptide may be used to deliver the nucleic acid construct according to the present invention. The peptide must have a cationic charge to electrostatically interact with the anionic phosphate group of the nucleic acid and may include a positively charged amino acid to electrostatically interact with the phosphate group. Specific details of peptides usable for mRNA delivery are disclosed in AIMS Biophysics, 7(4): 323-338, which is incorporated herein by reference.

The peptide usable for delivering the nucleic acid construct may include protamine. The protamine is a small, cationic, arginine-rich nucleoprotein that contributes to the stability of DNA during spermatogenesis in the testis. Protamine stabilizes mRNA molecules and thus efficiently delivers the same. US 9352028 B specifically discloses a protamine-mRNA complex, which is incorporated herein by reference.

Cell-penetrating peptides (CPPs) may also be promising cationic molecules for delivery of nucleic acid constructs. Non-limiting examples of such cationic molecules include, but are not limited to, amphipathic CPPs such as the arginine-rich RALA peptide (WEARLARALARALARHLARALARALRACEA: SEQ ID NO. 117), LAH4 (KKALLALALHHLAHLALHLALALKKA: SEQ ID NO. 118), and LAH4-L1 (KKALLAHALHLLALLALHLAHALKKA: SEQ ID NO. 119).

If desired, peptides may be contained in addition to the liposomes or LNPs for mRNA delivery. These peptides may provide nucleic acid packaging functions and protect DNA or RNA from intracellular or extracellular degradation. Examples of such peptides are specifically disclosed in US 2021/0170046 A, which is incorporated herein by reference, but is not limited thereto.

The pharmaceutical composition according to the present invention may comprise a pharmaceutically effective amount of the nucleic acid construct alone, or may further comprise at least one pharmaceutically acceptable carrier, excipient or diluent, in addition to the nucleic acid construct. The term "pharmaceutically effective amount" refers to an amount sufficient to prevent, ameliorate, and treat symptoms of a target disease.

The term "pharmaceutically acceptable" used herein means being physiologically acceptable without causing ordinary allergic reactions such as gastrointestinal disorders or dizziness or similar reactions thereto when administration to humans. Examples of the carrier, excipient and diluent include lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinylpyrrolidone, water, methyl hydroxybenzoate, propyl hydroxybenzoate, talc, magnesium stearate, and mineral oils. In addition, the pharmaceutical composition may further comprise fillers, anti-aggregating agents, lubricants, wetting agents, flavoring agents, emulsifying agents, preservatives and the like.

As used herein, the term "carrier" is defined as a substance that facilitates the addition of the nucleic acid construct to cells or tissues. For example, dimethyl sulfoxide (DMSO) is a commonly used carrier that facilitates the introduction of many organic compounds into the cells or tissues of living organisms.

The term "diluent" is defined as a compound that stabilizes the biologically active form of the target compound and is diluted in water to dissolve the compound. Salts dissolved in buffer solutions are used as diluents in the art. A commonly used buffer solution is phosphate-buffered saline because it mimics the salt state of human fluids. Because buffer salts can control the pH of a solution at low concentrations, buffer diluents rarely alter the biological activity of the compound.

The pharmaceutical composition comprising the nucleic acid construct according to the present invention may be administered to a patient on its own or as a pharmaceutical composition mixed with other active ingredients, such as in combination therapy, or with suitable carriers or excipients.

The pharmaceutical composition of the present invention may be administered via various routes, including oral, transdermal, subcutaneous, intravenous, or intramuscular routes. The dosage of the active ingredient may be appropriately selected based on various factors, such as the route of administration, the patient's age, gender, weight, and severity of the condition.

The pharmaceutical composition suitable for use in the present invention includes a composition comprising the nucleic acid construct encoding the active ingredient in an amount effective to achieve the intended purpose. More specifically, a therapeutically effective amount refers to an amount of the compound effective to prolong the survival of a subject to be treated, or to prevent, alleviate, or mitigate the symptoms of a disease. The therapeutically effective amount is determined within the capabilities of those skilled in the art, particularly in terms of the detailed disclosure provided herein.

As used herein, the term "subject" refers to a mammal suffering from or at risk of a condition or disease that may be alleviated, suppressed, or treated by administering the nucleic acid construct according to the present invention, preferably a human.

The dosage of the nucleic acid construct of the present invention administered to human may vary depending on the age, weight, gender, administration form, health status, and disease severity of patients.

The toxicity and therapeutic efficacy of compositions comprising the nucleic acid constructs described herein may be determined by standard pharmaceutical procedures in cell cultures or experimental animals, for example, to determine the LD₅₀ (lethal dose for 50% of a population), ED₅₀ (dose that has a therapeutic effect for 50% of a population), and IC₅₀ (dose that has a therapeutic inhibitory effect for 50% of a population). The dose ratio between toxic and therapeutic effects is the therapeutic index, which may be expressed as the ratio of the LD₅₀ to the ED₅₀ (or IC₅₀). Compounds that exhibit a high therapeutic index are preferred. Data obtained from these cell culture assays may be used to determine a range of doses for use in humans. The dosage or application amount of such compounds preferably falls within the range of circulating concentrations that include ED₅₀ (or IC₅₀) with little or no toxicity.

As used herein, the term "administration" refers to an action of introducing the pharmaceutical composition of the present invention into a subject by any appropriate method, and the route of administration may be through various routes, such as oral or parenteral, as long as it can reach the target tissue.

The pharmaceutical composition of the present invention may be administered via any conventional route as long as it can reach the target tissue. The pharmaceutical composition of the present invention is not particularly limited thereto, but may be administered intraperitoneally, intravenously, intramuscularly, subcutaneously, intradermally, orally, intranasally, intrapulmonary, or rectally, depending on the intended purpose. In addition, the pharmaceutical composition according to the present invention may be administered using any device capable of delivering the active substance to target cells.

In addition, the pharmaceutical composition of the present invention may be administered alone or in combination with other therapeutics. In this case, the composition may be administered sequentially or simultaneously with conventional therapeutics. In addition, the pharmaceutical composition may be administered in single or multiple doses. Taking into consideration these factors, it is important to administer the pharmaceutical composition in the minimum amount sufficient to achieve maximum efficacy without side effects.

The pharmaceutical composition according to the present invention may be formulated as an injectable formulation such as an aqueous solution, suspension, emulsion, or the like, but is not limited thereto, and is preferably provided in a lyophilized formulation. To prepare the lyophilized formulation, a method commonly known in the art to which the present invention pertains may be used and a stabilizer for lyophilization may be added. Furthermore, the composition may be preferably formulated depending on each disease or ingredient using an appropriate method in the art or a method disclosed in Remington's Pharmaceutical Science (Mack Publishing Company, Easton PA).

Hereinafter, the present invention will be described in more detail with reference to the following examples. However, it will be obvious to those skilled in the art that the following examples are provided only for illustration of the present invention and should not be construed as limiting the scope of the present invention.

### Example 1: Establishment of structure for protecting poly(A) end of mRNA

To protect poly(A) from exonuclease (deadenylase), proteins binding to the terminal region or structural formation should be used to minimize exposure. However, unlike proteins, mRNA is composed of nucleotides. Therefore, structural formation may be possible through unintended complementary and upon intracellular penetration, TLR3 (Sergio Linares-Fernandez, et al., Trends Mol Med. 2020 Mar;26(3):311-323) (recognizing a 35-nucleotide complementary binding sequence) is highly likely to halve its efficacy as an innate immune response mechanism. Therefore, candidates were selected on structural sequences that have already been experimentally applied in humans. The selection was based on viral sequences (Hiroyasu Wakida et al., Biochem Biophys Res Commun. 2020 Jul 5; 527(4):993-999, Kathrin Leppek et al., Nat Commun. 2022 Mar 22; 13(1):1536). RNA viruses have evolved over time to possess sequences that evade the innate immune system and enable efficient protein expression within host human cells and thus are used in sequence regions that do not induce pathogenic effects.

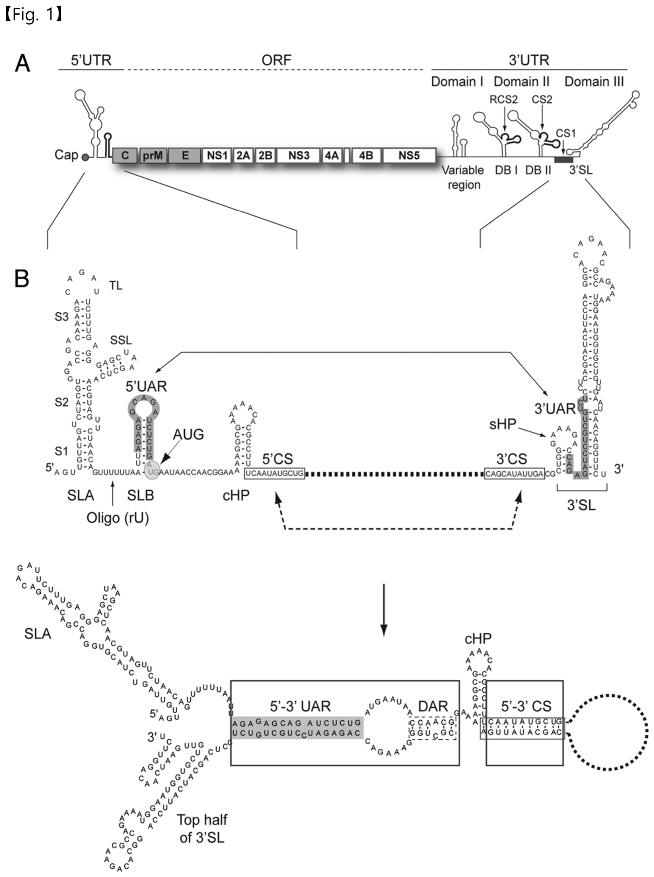

As a result, the Dengue virus (DENV) sequence was used. DENV has been reported to have evolved to possess a motif of three complementary binding sites (5'-3' UAR/DAR/CS) that enhance stability (FIG. 1; the three complementary binding sites (5'-3' UAR/DAR/CS) are indicated by the red box). Due to this structure, DENV is known to exhibit high viral replication and translation efficiency (Nestor G lglesias, Andrea V Gamarnik, RNA Biol. 2011 Mar-Apr;8(2):249-57).

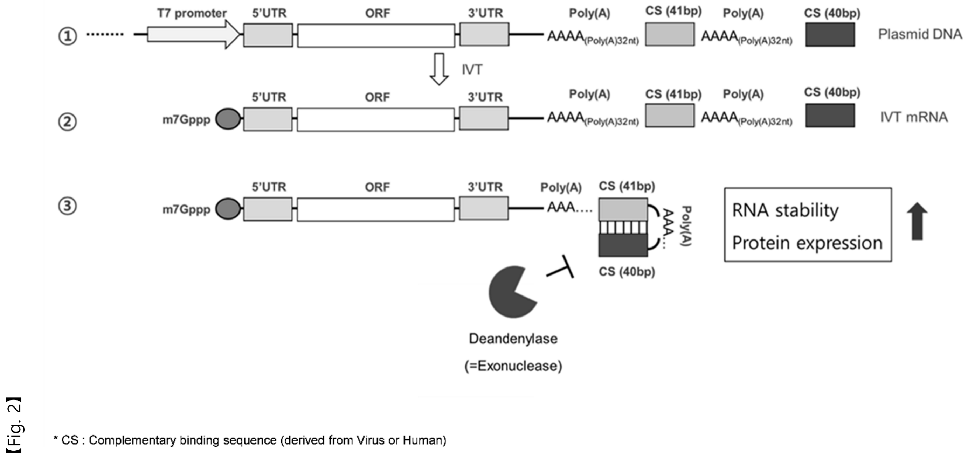

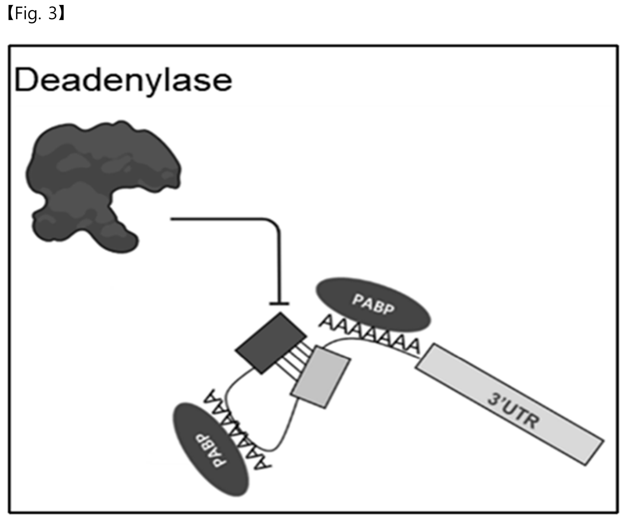

A platform construct with enhanced stability and translation efficiency was produced by combining the three structural sequences (5'-3' UAR/DAR/CS) described above, inserting the sequences into the middle and end of the poly(A) region, converting the unstable single-strand region of adenosine (A) terminal into double-strands and shielding the mRNA from exonuclease attack (FIG. 2). This construct is likely to block the access of exonucleases by forming double strands or secondary structures through complementary binding to the poly(A) end region (Stefan Bresson, David Tollervey, Open Biol. 2018 Mar;8(3):170270) (FIG. 3), and may function independently without interference with UTR and CDS sequences and thus may be used as a universally usable platform.

### Example 2: Design of mRNA construct and prediction of secondary structure

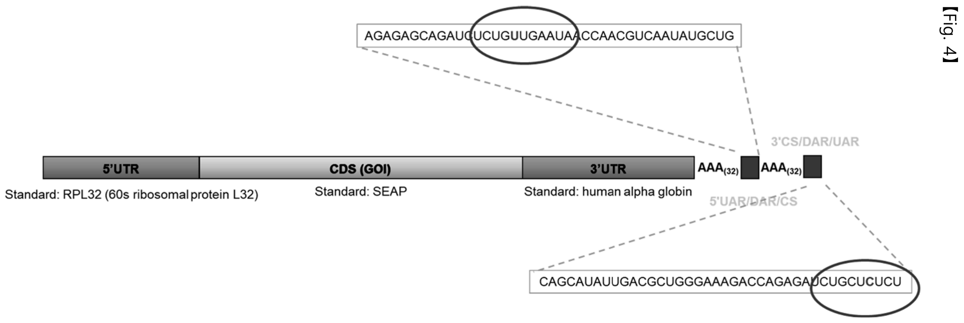

In the DENV sequence, the UAR/DAR/CS regions are each divided into independent regions. In the present invention, the nucleotide sequences of the three regions containing the bulged structure were modified and linked to increase the probability of maintaining complementary binding while avoiding innate immune mechanisms (Table 1, FIG. 4).

**[Table 1]**

| 5-'UTR, 3'-UTR and GOI sequences used in the construct according to the present invention | | |
|---|---|---|
| **Region** | **Sequence (5'->3')** | **SEQ ID NO.** |
| **5' UTR** | GGCGCUGCCUACGGAGGUGGCAGCCAUCUCCUUCUCGGCAUC | 19 |
| **3'UTR** | GCCCGAUGGGCCUCCCAACGGGCCCUCCUCCCCUCCUUGCACCG | 20 |
| **GOI (SEAP)** | AUUGCUUGCUUGCUUGCUUGCUUGCUUGGGCCUUGAGGCU UACAGCUUCUUCCCUUGGGCAUUCAUUCCCAGUUUUGAGGAGGA GAACCCGGACUUUUCUGGAACCGCAGGCAGCCGAGGCCCUGGG UGCCGCCAAGAAGCUGCAGCCUGCACAGACAGCCGCCAAGAACC UCAUCAUCUUCCUGGGCGAUGGGAUGGGGGUGUCUACGGUGACA GCUGCCAGGAUCCUAAAAGGGCAGAAGAAGGACAAACUGGGGCC UGAGAUACCCCUGGCCAUGGACCGCUUCCCAUAUGUGGCUCUGU CCAAGACAUACAAUGUAGACAAACAUGUGCCAGACAGUGGAGCC ACAGCCACGGCCUACCUGUGCGGGGUCAAGGGCAACUUCCAGAC CAUUGGCUUGAGUGCAGCCGCCCGCUUUAACCAGUGCAACACGA CACGCGGCAACGAGGUCAUCUCCGUGAUGAAUCGGGCCAAGAAA GCAGGGAAGUCAGUGGGAGUGGUAACCACCACACGAGUGCAGCA CGCCUCGCCAGCCGGCACCUACGCCCACACGGUGAACCGCAACU GGUACUCGGACGCCGACGUGCCUGCCUCGGCCCGCCAGGAGGGG UGCCAGGACAUCGCUACGCAGCUCAUCUCCAACAUGGACAUUGA CGUGAUCCUAGGUGGAGGCCGAAAGUACAUGUUUCGCAUGGGAA CCCCAGACCCUGAGUACCCAGAUGACUACAGCCAAGGUGGGACC AGGCUGGACGGGAAGAAUCUGGUGCAGGAAUGGCUGGCGAAGCG CCAGGGUGCCCGGUAUGUGUGGAACCGCACUGAGCUCAUGCAGG CUUCCCUGGACCCGUCUGUGACCCAUCUCAUGGGUCUCUUUGAG CCUGGAGACAUGAAAUACGAGAUCCACCGAGACUCCACACUGGA CCCCUCCCUGAUGGAGAUGACAGAGGCUGCCCUGCGCCUGCUGA GCAGGAACCCCCGCGGCUUCUUCCUCUUCGUGGAGGGUGGUCGC AUCGACCAUGGUCAUCAUGAAAGCAGGGCUUACCGGGCACUGAC UGAGACGAUCAUGUUCGACGACGCCAUUGAGAGGGCGGGCCAGC UCACCAGCGAGGAGGACACGCUGAGCCUCGUCACUGCCGACCAC UCCCACGUCUUCUCCUUCGGAGGCUACCCCCUGCGAGGGAGCUC CAUCUUCGGGCUGGCCCCUGGCAAGGCCCGGGACAGGAAGGCCU ACACGGUCCUCCUAUACGGAAACGGUCCAGGCUAUGUGCUCAAG GACGGCGCCCGGCCGGAUGUUACCGAGAGCGAGAGCGGGAGCCC CGAGUAUCGGCAGCAGUCAGCAGUGCCCCUGGACGAAGAGACCC ACGCAGGCGAGGACGUGGCGGUGUUCGCGCGCGGCCCGCAGGCG CACCUGGUUCACGGCGUGCAGGAGCAGACCUUCAUAGCGCACGU CAUGGCCUUCGCCGCCUGCCUGGAGCCCUACACCGCCUGCGACC UGGCGCCCCCCGCCGGCACCACCGACGCCGCGCACCCGGGUUAC | 21 |
| | UCUAGAGUCGGGGCGGCCGGCCGCUUCGAGCAGACAUGA | |
| **GOI (GLuc)** | AUGGGAGUCAAAGUUCUGUUUGCCCUGAUCUGCAUCGCUGUGGC CGAGGCCAAGCCCACCGAGAACAACGAAGACUUCAACAUCGUGG CCGUGGCCAGCAACUUCGCGACCACGGAUCUCGAUGCUGACCGC GGGAAGUUGCCCGGCAAGAAGCUGCCGCUGGAGGUGCUCAAAGA GAUGGAAGCCAAUGCCCGGAAAGCUGGCUGCACCAGGGGCUGUC UGAUCUGCCUGUCCCACAUCAAGUGCACGCCCAAGAUGAAGAAG UUCAUCCCAGGACGCUGCCACACCUACGAAGGCGACAAAGAGUC CGCACAGGGCGGCAUAGGCGAGGCGAUCGUCGACAUUCCUGAGA UUCCUGGGUUCAAGGACUUGGAGCCCAUGGAGCAGUUCAUCGCA CAGGUCGAUCUGUGUGUGGACUGCACAACUGGCUGCCUCAAAGG GCUUGCCAACGUGCAGUGUUCUGACCUGCUCAAGAAGUGGCUGC CGCAACGCUGUGCGACCUUUGCCAGCAAGAUCCAGGGCCAGGUG GACAAGAUCAAGGGGGCCGGUGGUGACUAA | 22 |
| **CS₁** | AGAGAGCAGAUCUCUGUUGAAUAACCAACGUCAAUAUGCUG | 1 |
| **CS₂** | CAGCAUAUUGACGCUGGGAAAGACCAGAGAUCUGCUCUCU | 10 |

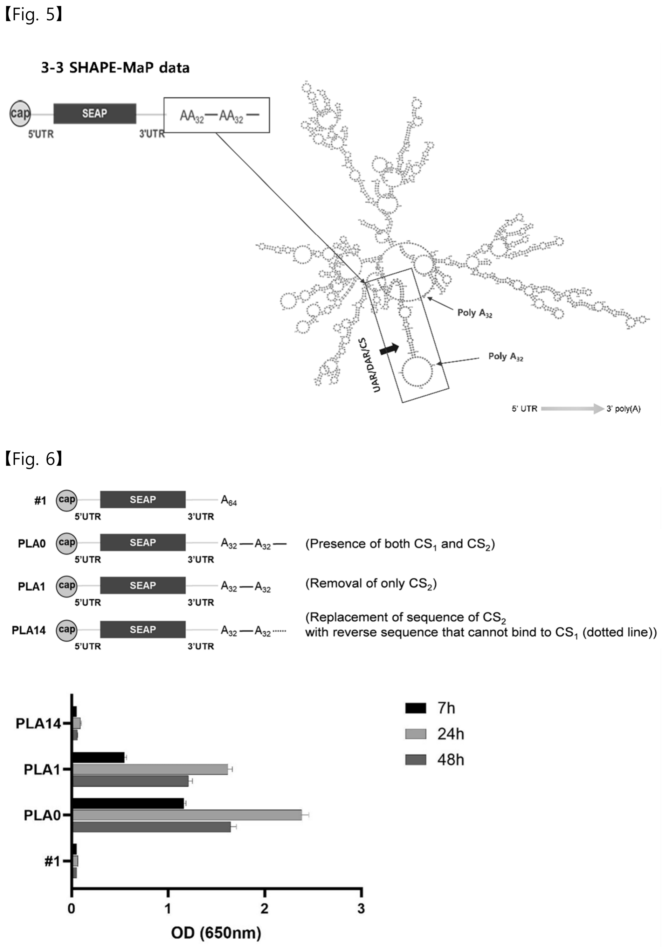

After designing the mRNA expression cassette construct, a SHAPE-Map assay to experimentally predict the structure of the mRNA synthesized *in vitro* was performed to identify that the complementary sequences of the modified DENV complementarily bound to each other form a secondary structure (FIG. 5; red box: secondary structure formation through complementary binding of CS₁ and CS₂).

### Example 3: Protein expression level test for in-vitro proof-of-concept

In addition to the construct shown in FIG. 5, pDNA of constructs (Table 2) to determine the effects of combinations of various numbers of poly(A) and secondary structure formation through complementary binding of CSs on increasing protein expression was synthesized, and mRNA was obtained through intravenous transfection (IVT) and transfected into HeLa cells. After transfection, media was harvested early (~7 hours) or late (24 hours & 48 hours), and secreted alkaline phosphatase (SEAP) activity was measured. The results are shown in FIG. 6.

**[Table 2]**

| Poly(A) region sequences of constructs | | |
|---|---|---|
| **Constructs** | **Poly(A) region sequences comprising CS₁ and CS₂ sequences (5' ->3')** | **SEQ ID NO.** |
| #1 | AAAAAAAAAAAAAAAAAAAAAAAAAAAAAAAAAAAAAAAAAAAAAAAAA AAAAAAAAAAAAAAAAAAAAA | 23 |
| PLA0 | AAAAAAAAAAAAAAAAAAAAAAAAAAAAAAAAGAGAGCAGAUCUC UGUUGAAUAACCAACGUCAAUAUGCUGAAAAAAAAAAAAAAAAAA AAAAAAAAAAAAAACAGCAUAUUGACGCUGGGAAAGACCAGAGAU CUGCUCUCU | 24 |
| PLA1 | AAAAAAAAAAAAAAAAAAAAAAAAAAAAAAAGAGAGCAGAUCUC UGUUGAAUAACCAACGUCAAUAUGCUGAAAAAAAAAAAAAAAAA AAAAAAAAAAAAAA | 25 |
| PLA14 | AAAAAAAAAAAAAAAAAAAAAAAAAAAAAAAGAGAGCAGAUCUC UGUUGAAUAACCAACGUCAAUAUGCUGAAAAAAAAAAAAAAAAA AAAAAAAAAAAAAAAAGAGAGCAGAUCUCUGGUCUUUCCCAGCGUC AAUAUGCUG | 26 |

| | | |
|---|---|---|
| (CS₁ and CS₂ sequences are lined) | | |

It was observed that the mRNA construct (PLA0) in the form of A₃₂-CS₁-A₃₂-CS₂, which has CS₁/CS₂ sequences that may complementarily bind to each other, exhibited increased SEAP activity compared to A₆₄ (control; #1) consisting of 64 poly(A) structures at the 3' end (FIG. 6). In order to determine whether or not the phenomenon is affected by the complementary binding of CS₁ and CS₂ sequences, constructs from which CS₂ was removed and modified were produced to conduct an experiment to compare SEAP activities. PLA1, which is expected to not form a complementary binding structure due to the absence of the last CS₂ sequence, and PLA14, which cannot complementarily bind to the CS₁ sequence because the CS₂ sequence is replaced with the reverse sequence of the existing sequence, were produced and the effects thereof was determined. The result showed that the protein expression efficiency of PLA1 was decreased compared to the protein expression efficiency of PLA0. Furthermore, the protein expression efficiency of PLA14 was similar to that of #1 (A₆₄).

Consequently, the SHAPE-Map assay showed that the complementary binding of CS₁ and CS₂ sequences can form a secondary structure (FIG. 5). This secondary structure, in turn, shields the poly(A) end, thereby enhancing the protein expression level of the corresponding mRNA (FIG. 6).

### Example 4: Search for combination of optimal number of poly(A) using complementary binding structure of present invention

To determine the combination of optimal number of poly(A) that maximizes protein expression efficiency from poly(A) in front of CS₁ (proximal poly(A)) and poly(A) between CS₁ and CS₂ (distal poly(A)), various combinations were tested.

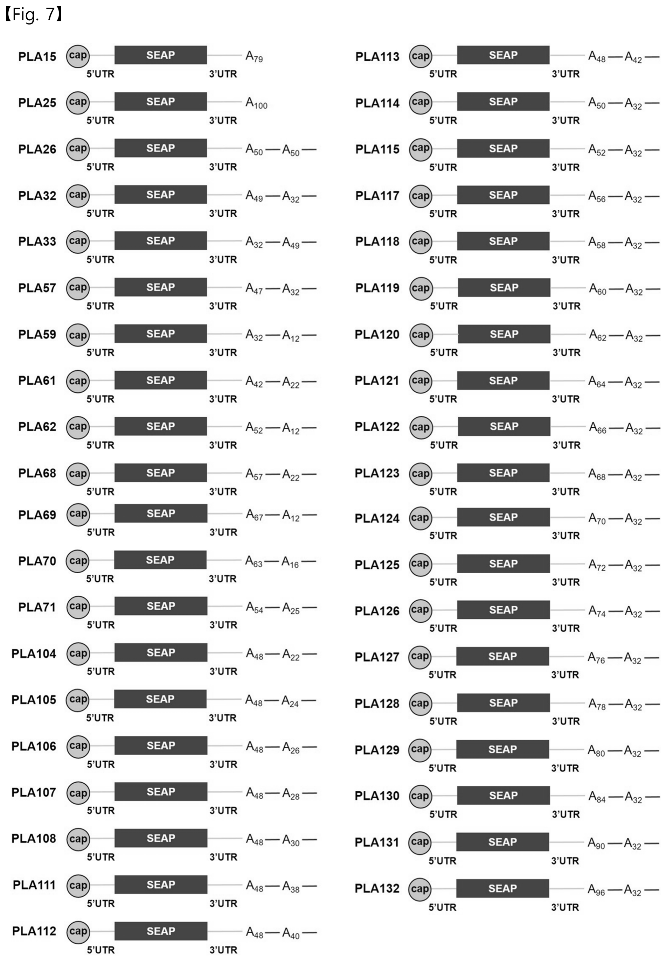

Previous reports have shown that total poly (A) sequence and poly(A) binding protein (PABP) that binds to the poly(A) sequence protect the poly(A) end from the attack of the deadenylase complex, function to increase RNA stability and induce protein translation initiation. Therefore, as the number of poly(A) to which PABP can bind increases, the protein expression efficiency may be expected to increase. However, recent reports have shown that there is a minimum number of poly(A) to which PABP binds (Michael W Webste, et al., Mol Cell. 2018 Jun 21;70(6):1089-1100.e8; Seung Hwan Lee, et al., Nucleic Acids Res. 2014 Feb;42(4):2697-707). Therefore, based on the required minimum number of poly(A) to which PABP binds, the protein expression efficiency depending on various combinations of poly(A) numbers was verified (Table 3, FIG. 7). The present experiment aimed at identifying the optimal PABP binding combination along with the effective shielding of the poly(A) end region using CS₁ and CS₂ to provide stable protein expression.

**[Table 3]**

| Poly(A) region sequences of constructs | | |
|---|---|---|
| **Constructs** | **Poly(A) region sequences comprising CS₁ and CS₂ sequences (5'->3')** | **SEQ ID NO.** |
| PLA15 | AAAAAAAAAAAAAAAAAAAAAAAAAAAAAAAAAAAAAAAA AAAAAAAAAAAAAAAAAAAAAAAAAAAAAAAAAAAAA | 27 |
| PLA25 | AAAAAAAAAAAAAAAAAAAAAAAAAAAAAAAAAAAAAAAAAA | 28 |
| | AAAAAAAAAAAAAAAAAAAAAAAAAAAAAAAAAAAAAAAAAAAAA AAAAAAAAAAAAAAAAAAAA | |
| PLA26 | AAAAAAAAAAAAAAAAAAAAAAAAAAAAAAAAAAAAAAAA AAAAAAAAAGAGAGCAGAUCUCUGUUGAAUAACCAACGUC AAUAUGCUGAAAAAAAAAAAAAAAAAAAAAAAAAAAAAAA AAAAAAAAAAAAAAAAAACAGCAUAUUGACGCUGGGAAAGA CCAGAGAUCUGCUCUCU | 29 |
| PLA32 | AAAAAAAAAAAAAAAAAAAAAAAAAAAAAAAAAAAAAAAAA AAAAAAAAGAGAGCAGAUCUCUGUUGAAUAACCAACGUCAA UAUGCUGAAAAAAAAAAAAAAAAAAAAAAAAAAAAAAAACA GCAUAUUGACGCUGGGAAAGACCAGAGAUCUGCUCUCU | 30 |
| PLA33 | AAAAAAAAAAAAAAAAAAAAAAAAAAAAAAAAAGAGAGCAG AUCUCUGUUGAAUAACCAACGUCAAUAUGCUGAAAAAAAAA AAAAAAAAAAAAAAAAAAAAAAAAAAAAAAAAAAAAAAACA GCAUAUUGACGCUGGGAAAGACCAGAGAUCUGCUCUCU | 31 |
| PLA57 | AAAAAAAAAAAAAAAAAAAAAAAAAAAAAAAAAAAAAAAA AAAAAAGAGAGCAGAUCUCUGUUGAAUAACCAACGUCAAUA UGCUGAAAAAAAAAAAAAAAAAAAAAAAAAAAAAAAACAGC AUAUUGACGCUGGGAAAGACCAGAGAUCUGCUCUCU | 32 |
| PLA59 | AAAAAAAAAAAAAAAAAAAAAAAAAAAAAAAAAAAAAAAAAA AAAAAAGAGAGCAGAUCUCUGUUGAAUAACCAACGUCAAUA UGCUGAAAAAAAAAAAACAGCAUAUUGACGCUGGGAAAGAC CAGAGAUCUGCUCUCU | 33 |
| PLA61 | AAAAAAAAAAAAAAAAAAAAAAAAAAAAAAAAAAAAAAAAA AGAGAGCAGAUCUCUGUUGAAUAACCAACGUCAAUAUGCUG AAAAAAAAAAAAAAAAAAAAAACAGCAUAUUGACGCUGGGA AAGACCAGAGAUCUGCUCUCU | 34 |
| PLA62 | AAAAAAAAAAAAAAAAAAAAAAAAAAAAAAAAAAAAAAAA AAAAAAAAAAGAGAGCAGAUCUCUGUUGAAUAACCAACGU CAAUAUGCUGAAAAAAAAAAAACAGCAUAUUGACGCUGGGA AAGACCAGAGAUCUGCUCUCU | 35 |
| PLA68 | AAAAAAAAAAAAAAAAAAAAAAAAAAAAAAAAAAAAAAAAA AAAAAAAAAAAAAAAAGAGAGCAGAUCUCUGUUGAAUAACC AACGUCAAUAUGCUGAAAAAAAAAAAAAAAAAAAAAACAGC AUAUUGACGCUGGGAAAGACCAGAGAUCUGCUCUCU | 36 |
| PLA69 | AAAAAAAAAAAAAAAAAAAAAAAAAAAAAAAAAAAAAAAAA AAAAAAAAAAAAAAAAAAAAAAAAAAGAGAGCAGAUCUCUG UUGAAUAACCAACGUCAAUAUGCUGAAAAAAAAAAAACAGC AUAUUGACGCUGGGAAAGACCAGAGAUCUGCUCUCU | 37 |
| PLA70 | AAAAAAAAAAAAAAAAAAAAAAAAAAAAAAAAAAAAAAAAA AAAAAAAAAAAAAAAAAAAAAGAGAGCAGAUCUCUGUUGA AUAACCAACGUCAAUAUGCUGAAAAAAAAAAAAAAAACAGC AUAUUGACGCUGGGAAAGACCAGAGAUCUGCUCUCU | 38 |
| PLA71 | AAAAAAAAAAAAAAAAAAAAAAAAAAAAAAAAAAAAAAAA AAAAAAAAAAAAGAGAGCAGAUCUCUGUUGAAUAACCAAC GUCAAUAUGCUGAAAAAAAAAAAAAAAAAAAAAACAGC AUAUUGACGCUGGGAAAGACCAGAGAUCUGCUCUCU | 39 |
| PLA104 | AAAAAAAAAAAAAAAAAAAAAAAAAAAAAAAAAAAAAAAAA AAAAAAAAGAGAGCAGAUCUCUGUUGAAUAACCAACGUCAA UAUGCUGAAAAAAAAAAAAAAAAAAAAAACAGCAUAUUGAC GCUGGGAAAGACCAGAGAUCUGCUCUCU | 40 |
| PLA105 | AAAAAAAAAAAAAAAAAAAAAAAAAAAAAAAAAAAAAAAAAAAAA AAAAAAAAGAGAGCAGAUCUCUGUUGAAUAACCAACGUCAA UAUGCUGAAAAAAAAAAAAAAAAAAAAAAAAAACAGCAUAUUG ACGCUGGGAAAGACCAGAGAUCUGCUCUCU | 41 |
| PLA106 | AAAAAAAAAAAAAAAAAAAAAAAAAAAAAAAAAAAAAAAA AAAAAAAAGAGAGCAGAUCUCUGUUGAAUACCAACGUCAA UAUGCUGAAAAAAAAAAAAAAAAAAAAAAAAACAGCAUAU UGACGCUGGGAAAGACCAGAGAUCUGCUCUCU | 42 |
| PLA107 | AAAAAAAAAAAAAAAAAAAAAAAAAAAAAAAAAAAAAAAA AAAAAAAGAGAGCAGAUCUCUGUUGAAUAACCAACGUCAA UAUGCUGAAAAAAAAAAAAAAAAAAAAAAAAAAACAGCAU AUUGACGCUGGGAAAGACCAGAGAUCUGCUCUCU | 43 |
| PLA108 | AAAAAAAAAAAAAAAAAAAAAAAAAAAAAAAAAAAAAAAA AAAAAAAAGAGAGCAGAUCUCUGUUGAAUAACCAACGUCAA UAUGCUGAAAAAAAAAAAAAAAAAAAAAAAAAAAAAACAGC AUAUUGACGCUGGGAAAGACCAGAGAUCUGCUCUCU | 44 |
| PLA111 | AAAAAAAAAAAAAAAAAAAAAAAAAAAAAAAAAAAAAAAAA AAAAAAAAGAGAGCAGAUCUCUGUUGAAUAACCAACGUCAA UAUGCUGAAAAaaAAAAAAAAAAAAAAAAAAAAAAAAAAAA AAAACAGCAUAUUGACGCUGGAAAGACCAGAGAUCUGCUC UCU | 45 |
| PLA112 | AAAAAAAAAAAAAAAAAAAAAAAAAAAAAAAAAAAAAAAA AAAAAAAAGAGAGCAGAUCUCUGUUGAAUAACCAACGUCAA UAUGCUGAAAAAAaaAAAAAAAAAAAAAAAAAAAAAAAAAA AAAAAACAGCAUAUUGACGCUGGGAAAGACCAGAGAUCUGC UCUCU | 46 |
| PLA113 | AAAAAAAAAAAAAAAAAAAAAAAAAAAAAAAAAAAAAAAA AAAAAAAGAGAGCAGAUCUCUGUUGAAUAACCAACGUCAA UAUGCUGAAAAAAAAaaAAAAAAAAAAAAAAAAAAAAAAA AAAAAAACAGCAUAUUGACGCUGGGAAAGACCAGAGAUCU GCUCUCU | 47 |
| PLA114 | AAAAAAAAAAAAAAAAAAAAAAAAAAAAAAAAAAAAAAAAA AAAAAAAAAAGAGAGCAGAUCUCUGUUGAAUAACCAACGUC AAUAUGCUGAAAAAAAAAAAAAAAAAAAAAAAAAAAAAAAA CAGCAUAUUGACGCUGGGAAAGACCAGAGAUCUGCUCUCU | 48 |
| PLA115 | AAAAAAAAAAAAAAAAAAAAAAAAAAAAAAAAAAAAAAAA AAAAAAAAAAAAAGAGAGCAGAUCUCUGUUGAAUAACCAACG UCAAUAUGCUGAAAAAAAAAAAAAAAAAAAAAAAAAAAAA AAACAGCAUAUUGACGCUGGGAAAGACCAGAGAUCUGCUCUC U | 49 |
| PLA117 | AAAAAAAAAAAAAAAAAAAAAAAAAAAAAAAAAAAAAAAA AAAAAAAAAAAAAAAGAGAGCAGAUCUCUGUUGAAUAACC AACGUCAAUAUGCUGAAAAAAAAAAAAAAAAAAAAAAAAA | 50 |
| | AAAAAACAGCAUAUUGACGCUGGGAAAGACCAGAGAUCUGC UCUCU | |
| PLA118 | AAAAAAAAAAAAAAAAAAAAAAAAAAAAAAAAAAAAAAAA AAAAAAAAAAAAAAAAGAGAGCAGAUCUCUGUUGAAUAAC CAACGUCAAUAUGCUGAAAAAAAAAAAAAAAAAAAAAAAA AAAAAAAACAGCAUAUUGACGCUGGGAAAGACCAGAGAUCUG CUCUCU | 51 |
| PLA119 | AAAAAAAAAAAAAAAAAAAAAAAAAAAAAAAAAAAAAAAA AAAAAAAAAAAAAAAAAAGAGAGCAGAUCUCUGUUGAAUA ACCAACGUCAAUAUGCUGAAAAAAAAAAAAAAAAAAAAAA AAAAAAAAAACAGCAUAUUGACGCUGGGAAAGACCAGAGAUC UGCUCUCU | 52 |
| PLA120 | AAAAAAAAAAAAAAAAAAAAAAAAAAAAAAAAAAAAAAAA AAAAAAAAAAAAAAAAAAAAGAGAGCAGAUCUCUGUUGAA UAACCAACGUCAAUAUGCUGAAAAAAAAAAAAAAAAAAAA AAAAAAAAAAAACAGCAUAUUGACGCUGGGAAAGACCAGAGA UCUGCUCUCU | 53 |
| PLA121 | AAAAAAAAAAAAAAAAAAAAAAAAAAAAAAAAAAAAAAAAA AAAAAAAAAAAAAAAAAAAAAAAGAGAGCAGAUCUCUGUU GAAUAACCAACGUCAAUAUGCUGAAAAAAAAAAAAAAAAAA AAAAAAAAAAAAAACAGCAUAUUGACGCUGGGAAAGACCAG AGAUCUGCUCUCU | 54 |
| PLA122 | AAAAAAAAAAAAAAAAAAAAAAAAAAAAAAAAAAAAAAAA AAAAAAAAAAAAAAAAAAAAAAAAAGAGAGCAGAUCUCUG UUGAAUAACCAACGUCAAUAUGCUGAAAAAAAAAAAAAAA AAAAAAAAAAAAAAAAACAGCAUAUUGACGCUGGGAAAGACC AGAGAUCUGCUCUCU | 55 |
| PLA123 | AAAAAAAAAAAAAAAAAAAAAAAAAAAAAAAAAAAAAAAA AAAAAAAAAAAAAAAAAAAAAAAAAAAAGAGCAGAUCUC UGUUGAAUAACCAACGUCAAUAUGCUGAAAAAAAAAAAAAA AAAAAAAAAAAAAAAAAACAGCAUAUUGACGCUGGGAAAGA CCAGAGAUCUGCUCUCU | 56 |
| PLA124 | AAAAAAAAAAAAAAAAAAAAAAAAAAAAAAAAAAAAAAAA AAAAAAAAAAAAAAAAAAAAAAAAAAAAAGAGAGCAGAUC UCUGUUGAAUAACCAACGUCAAUAUGCUGAAAAAAAAAAAA AAAAAAAAAAAAAAAAAAAACAGCAUAUUGACGCUGGGAAA GACCAGAGAUCUGCUCUCU | 57 |
| PLA125 | AAAAAAAAAAAAAAAAAAAAAAAAAAAAAAAAAAAAAAAAA AAAAAAAAAAAAAAAAAAAAAAAAAAAAAAAAAAGAGCAGA UCUCUGUUGAAUAACCAACGUCAAUAUGCUGAAAAAAAAAA AAAAAAAAAAAAAAAAAAAAAACAGCAUAUUGACGCUGGGA AAGACCAGAGAUCUGCUCUCU | 58 |
| PLA126 | AAAAAAAAAAAAAAAAAAAAAAAAAAAAAAAAAAAAAAAAA AAAAAAAAAAAAAAAAAAAAAAAAAAAAAAAAAAAAGAGAGCA GAUCUCUGUUGAAUAACCAACGUCAAUAUGCUGAAAAAAAA AAAAAAAAAAAAAAAAAAAAAAAACAGCAUAUUGACGCUGG GAAAGACCAGAGAUCUGCUCUCU | 59 |
| PLA127 | AAAAAAAAAAAAAAAAAAAAAAAAAAAAAAAAAAAAAAAAA AAAAAAAAAAAAAAAAAAAAAAAAAAAAAAAAAAAAGAGAG CAGAUCUCUGUUGAAUAACCAACGUCAAUAUGCUGAAAAAA AAAAAAAAAAAAAAAAAAAAAAAAAACAGCAUAUUGACGCU GGGAAAGACCAGAGAUCUGCUCUCU | 60 |
| PLA128 | AAAAAAAAAAAAAAAAAAAAAAAAAAAAAAAAAAAAAAAA AAAAAAAAAAAAAAAAAAAAAAAAAAAAAAAAAAAAAAAGAG AGCAGAUCUCUGUUGAAUAACCAACGUCAAUAUGCUGAAAA AAAAAAAAAAAAAAAAAAAAAAAAAAAACAGCAUAUUGACG CUGGGAAAGACCAGAGAUCUGCUCUCU | 61 |
| PLA129 | AAAAAAAAAAAAAAAAAAAAAAAAAAAAAAAAAAAAAAAAA AAAAAAAAAAAAAAAAAAAAAAAAAAAAAAAAAAAAAAAAG AGAGCAGAUCUCUGUUGAAUAACCAACGUCAAUAUGCUGAA AAAAAAAAAAAAAAAAAAAAAAAAAAAAAACAGCAUAUUGA CGCUGGGAAAGACCAGAGAUCUGCUCUCU | 62 |
| PLA130 | AAAAAAAAAAAAAAAAAAAAAAAAAAAAAAAAAAAAAAAAA AAAAAAAAAAAAAAAAAAAAAAAAAAAAAAAAAAAAAAAAA AAAGAGAGCAGAUCUCUGUUGAAUAACCAACGUCAAUAUGC UGAAAAAAAAAAAAAAAAAAAAAAAAAAAAAAAACAGCAUA UUGACGCUGGGAAAGACCAGAGAUCUGCUCUCU | 63 |
| PLA131 | AAAAAAAAAAAAAAAAAAAAAAAAAAAAAAAAAAAAAAAAA AAAAAAAAAAAAAAAAAAAAAAAAAAAAAAAAAAAAAAAAA AAAAAAAAAGAGAGCAGAUCUCUGUUGAAUAACCAACGUCA AUAUGCUGAAAAAAAAAAAAAAAAAAAAAAAAAAAAAAAAC AGCAUAUUGACGCUGGGAAAGACCAGAGAUCUGCUCUCU | 64 |
| PLA132 | AAAAAAAAAAAAAAAAAAAAAAAAAAAAAAAAAAAAAAAA AAAAAAAAAAAAAAAAAAAAAAAAAAAAAAAAAAAAAAAA AAAAAAAAAAAAAAGAGAGCAGAUCUCUGUUGAAUAACCA ACGUCAAUAUGCUGAAAAAAAAAAAAAAAAAAAAAAAAAA AAAAAACAGCAUAUUGACGCUGGGAAAGACCAGAGAUCUGCU CUCU | 65 |

| | | |
|---|---|---|
| (CS₁ and CS₂ sequences are lined) | | |

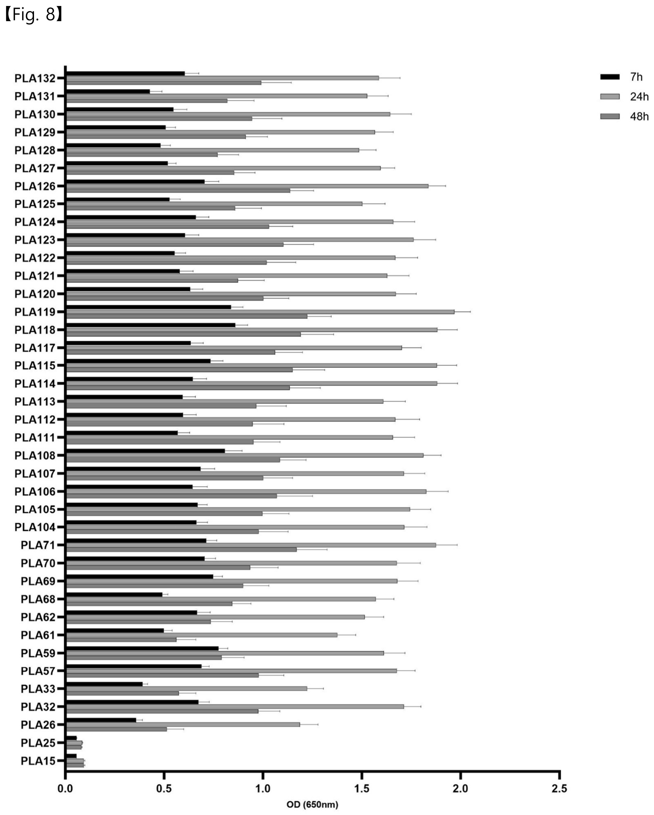

As a result, it was confirmed that protein expression efficiency was enhanced when the number of proximal poly(A)s at the very front and the number of distal poly(A)s existing between CS₁ and CS₂ in the A-CS₁-A-CS₂ structure was 12 or more (FIG. 8).

### Example 5: Exploration of additional constructs capable of enhancing protein expression using complementary binding sequences

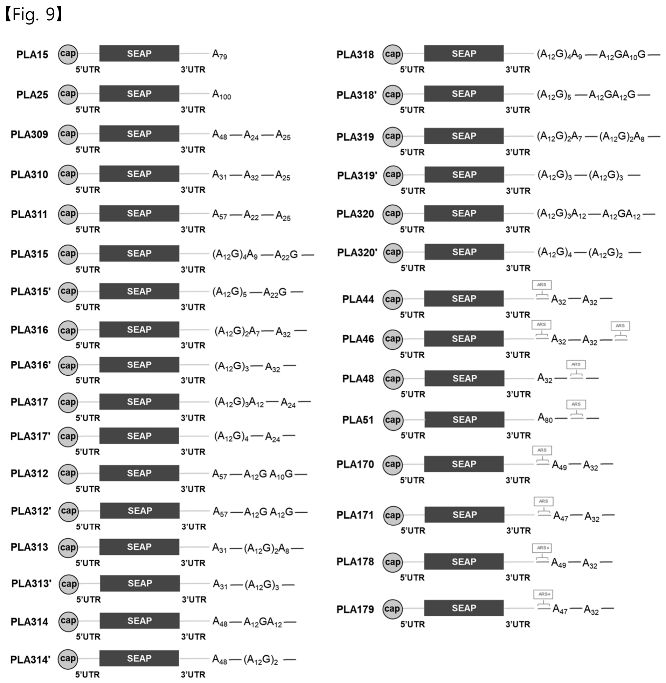

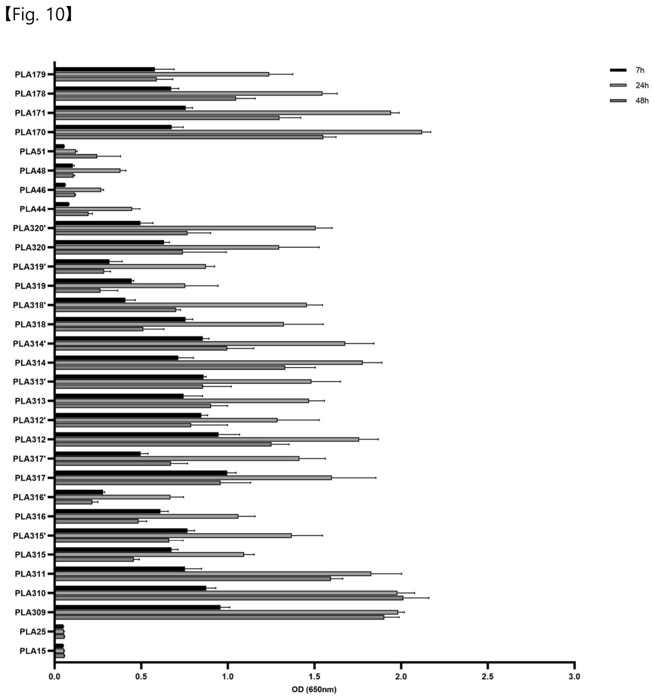

As shown in Table 4 and FIG. 9, various combinations of constructs were explored using the CS₁ and CS₂ sequences. The result showed that the CS₁ and CS₂ constructs may be applied to various combinations (FIG. 10) .

**[Table 4]**

| Poly(A) region sequences of constructs | | |
|---|---|---|
| **Constructs** | **Poly (A) region sequences comprising CS₁ and CS₂ sequences (5'->3')** | **SEQ ID NO.** |
| PLA15 | AAAAAAAAAAAAAAAAAAAAAAAAAAAAAAAAAAAAAAAAAAAAAAAAAA AAAAAAAAAAAAAAAAAAAAAAAAAAAAAAAAAAAAAAA | 27 |
| PLA25 | AAAAAAAAAAAAAAAAAAAAAAAAAAAAAAAAAAAAAAAAAAAAA AAAAAAAAAAAAAAAAAAAAAAAAAAAAAAAAAAAAAAAAAAAAA AAAAAAAAAA | 28 |
| PLA309 | AAAAAAAAAAAAAAAAAAAAAAAAAAAAAAAAAAAAAAAAAAAAA AAAAGAGAGCAGAUCUCUGUUGAAUAACCAACGUCAAUAUGCUGA AAAAAAAAAAAAAAAAAAAAAAAACAGCAUAUUGACGCUGGAAAG ACCAGAGAUCUGCUCUCUAAAAAAAAAAAAAAAAAAAAAAAAAA | 66 |
| PLA310 | AAAAAAAAAAAAAAAAAAAAAAAAAAAAAAAAAGAGAGCAGAUCUC UGUUGAAUAACCAACGUCAAUAUGCUGAAAAAAAAAAAAAAAAAAA AAAAAAAAAAAAACAGCAUAUUGACGCUGGGAAAGACCAGAGAU CUGCUCUCUAAAAAAAAAAAAAAAAAAAAAAAAA | 67 |
| PLA311 | AAAAAAAAAAAAAAAAAAAAAAAAAAAAAAAAAAAAAAAAAAAA AAAAAAAAAAAGAGAGCAGAUCUCUGUUGAAUAACCAACGUCAA UAUGCUGAAAAAAAAAAAAAAAAAAAAAACAGCAUAUUGACGCUG GGAAAGACCAGAGAUCUGCUCUCUAAAAAAAAAAAAAAAAAAAA AAA | 68 |
| PLA315 | AAAAAAAAAAAGAAAAAAAAAAAAGAAAAAAAAAAAGAAAAAA AAAAAAGAAAAAAAAAAGAGAGCAGAUCUCUGUUGAAUAACCAACG UCAAUAUGCUGAAAAAAAAAAAAAAAAAAAAAAGCAGCAUAUUGA CGCUGGGAAAGACCAGAGAUCUGCUCUCU | 69 |
| PLA315' | AAAAAAAAAAAAAAGAAAAAAAAAAAAAAAGAAAAAAAAAAAAAAAGAAAAAAA AAAAAAGAAAAAAAAAAAAAAAGGAGAGCAGAUCUCUGUUGAAUAACC AACGUCAAUAUGCUGAAAAAAAAAAAAAAAAAAAAAAAAAAGCAGCAUA UUGACGCUGGGAAAGACCAGAGAUCUGCUCUCU | 70 |
| PLA316 | AAAAAAAAAAAAGAAAAAAAAAAAAGAAAAAAAAGAGAGCAGAUC UCUGUUGAAUAACCAACGUCAAUAUGCUGAAAAAAAAAAAAAAA AAAAAAAAAAAAAAAAACAGCAUAUUGACGCUGGGAAAGACCAGAG AUCUGCUCUCU | 71 |
| PLA316' | AAAAAAAAAAAAGAAAAAAAAAAAAGAAAAAAAAAAAAGAGAGAG CAGAUCUCUGUUGAAUAACCAACGUCAAUAUGCUGAAAAAAAAA AAAAAAAAAAAAAAAAAAAAAAAACAGCAUAUUGACGCUGGAAAGA CCAGAGAUCUGCUCUCU | 72 |
| PLA317 | AAAAAAAAAAAAGAAAAAAAAAAAAGAAAAAAAAAAAAGAAAAAA AAAAAAAGAGAGCAGAUCUCUGUUGAAUAACCAACGUCAAUAUGC UGAAAAAAAAAAAAAAAAAAAAAAAACAGCAUAUUGACGCUGGGA AAGACCAGAGAUCUGCUCUCU | 73 |
| PLA317' | AAAAAAAAAAAAGAAAAAAAAAAAAGAAAAAAAAAAAAGAAAAAA AAAAAAGAGAGAGCAGAUCUCUGUUGAAUAACCAACGUCAAUAUG CUGAAAAAAAAAAAAAAAAAAAAAAAACAGCAUAUUGACGCUGGG AAAGACCAGAGAUCUGCUCUCU | 74 |
| PLA312 | AAAAAAAAAAAAAAAAAAAAAAAAAAAAAAAAAAAAAAAAAAAAAAAAAA AAAAAAAAAAAAAGAGAGCAGAUCUCUGUUGAAUAACCAACGUCAA UAUGCUGAAAAAAAAAAAAAGAAAAAAAAAAAGCAGCAUAUUGACGC UGGGAAAGACCAGAGAUCUGCUCUCU | 75 |
| PLA312' | AAAAAAAAAAAAAAAAAAAAAAAAAAAAAAAAAAAAAAAAAAAAAAAAAA AAAAAAAAAAAAAGAGAGCAGAUCUCUGUUGAAUAACCAACGUCAA UAUGCUGAAAAAAAAAAAAGAAAAAAAAAAAAGCAGCAUAUUGAC GCUGGGAAAGACCAGAGAUCUGCUCUCU | 76 |
| PLA313 | AAAAAAAAAAAAAAAAAAAAAAAAAAAAAAAAAGAGAGCAGAUCUC UGUUGAAUAACCAACGUCAAUAUGCUGAAAAAAAAAAAAGAAAAA AAAAAAAGAAAAAAAACAGCAUAUUGACGCUGGGAAAGACCAGAG AUCUGCUCUCU | 77 |
| PLA313' | AAAAAAAAAAAAAAAAAAAAAAAAAAAAAAAAAGAGAGCAGAUCUC UGUUGAAUAACCAACGUCAAUAUGCUGAAAAAAAAAAAAAGAAAAA AAAAAAAGAAAAAAAAAAAAAAGCAGCAUAUUGACGCUGGAAAGAC CAGAGAUCUGCUCUCU | 78 |
| PLA314 | AAAAAAAAAAAAAAAAAAAAAAAAAAAAAAAAAAAAAAAAAAAAAAAAAA AAAAGAGAGCAGAUCUCUGUUGAAUAACCAACGUCAAUAUGCUGA AAAAAAAAAAAAGAAAAAAAAAAAAACAGCAUAUUGACGCUGGGAAA GACCAGAGAUCUGCUCUCU | 79 |
| PLA314' | AAAAAAAAAAAAAAAAAAAAAAAAAAAAAAAAAAAAAAAAAAAAAAAAAA AAAAGAGAGCAGAUCUCUGUUGAAUAACCAACGUCAAUAUGCUGA AAAAAAAAAAAAGAAAAAAAAAAAAAAGCAGCAUAUUGACGCUGGGAA AGACCAGAGAUCUGCUCUCU | 80 |
| PLA318 | AAAAAAAAAAAAGAAAAAAAAAAAAGAAAAAAAAAAAAGAAAAAA AAAAAAGAAAAAAAAAAAAGAGAGCAGAUCUCUGUUGAAUAACCAACG UCAAUAUGCUGAAAAAAAAAAAAGAAAAAAAAAAAAGCAGCAUAUUG ACGCUGGGAAAGACCAGAGAUCUGCUCUCU | 81 |
| PLA318' | AAAAAAAAAAAAGAAAAAAAAAAAAAGAAAAAAAAAAAAAGAAAAAA AAAAAAGAAAAAAAAAAAAAGAGAGCAGAUCUCUGUUGAAUAACCA ACGUCAAUAUGCUGAAAAAAAAAAAAGAAAAAAAAAAAAAGCAGCA UAUUGACGCUGGGAAAGACCAGAGAUCUGCUCUCU | 82 |
| PLA319 | AAAAAAAAAAAAAGAAAAAAAAAAAAAAGAAAAAAAAAGAGAGCAGAUC UCUGUUGAAUAACCAACGUCAAUAUGCUGAAAAAAAAAAAAAGAAA AAAAAAAAAGAAAAAAAAACAGCAUAUUGACGCUGGAAAGACCAG AGAUCUGCUCUCU | 83 |
| PLA319' | AAAAAAAAAAAAGAAAAAAAAAAAAGAAAAAAAAAAAAGAGAGAG CAGAUCUCUGUUGAAUAACCAACGUCAAUAUGCUGAAAAAAAAAA AAGAAAAAAAAAAAAGAAAAAAAAAAAAGCAGCAUAUUGACGCUG GGAAAGACCAGAGAUCUGCUCUCU | 84 |
| PLA320 | AAAAAAAAAAAAGAAAAAAAAAAAAGAAAAAAAAAAAAGAAAAAA AAAAAAAGAGAGCAGAUCUCUGUUGAAUAACCAACGUCAAUAUGC UGAAAAAAAAAAAAGAAAAAAAAAAAACAGCAUAUUGACGCUGGG AAAGACCAGAGAUCUGCUCUCU | 85 |
| PLA320' | AAAAAAAAAAAAGAAAAAAAAAAAAGAAAAAAAAAAAAGAAAAAA AAAAAAGAGAGAGCAGAUCUCUGUUGAAUAACCAACGUCAAUAUG CUGAAAAAAAAAAAAGAAAAAAAAAAAAGCAGCAUAUUGACGCUG GGAAAGACCAGAGAUCUGCUCUCU | 86 |
| PLA44 | AAAAAAUCCAAAAAAAAUCUAAAAAAAUCUUUUAAAAAACCCCAA AAAAAUUUACAAAAAAGGAUCCAAGAUUAAUAAAAAAAAAAAA AAAAAAAAAAAAAAAAAAAAGAGAGCAGAUCUCUGUUGAAUAACCAA CGUCAAUAUGCUGAAAAAAAAAAAAAAAAAAAAAAAAAAAAAAAA CAGCAUAUUGACGCUGGGAAAGACCAGAGAUCUGCUCUCU | 87 |
| PLA46 | AAAAAAUCCAAAAAAAAUCUAAAAAAAUCUUUUAAAAAACCCCAA AAAAAUUUACAAAAAAGGAUCCAAGAUUAAUAAAAAAAAAAAAA AAAAAAAAAAAAAAAAAAAGAGAGCAGAUCUCUGUUGAAUAACCAA CGUCAAUAUGCUGAAAAAAAAAAAAAAAAAAAAAAAAAAAAAAAA CAGCAUAUUGACGCUGGGAAAGACCAGAGAUCUGCUCUCUAAAAA AUCCAAAAAAAAUCUAAAAAAAUCUUUAAAAAACCCAAAAAAA UUUACAAAAAAGGAUCCA | 88 |
| PLA48 | AAAAAAAAAAAAAAAAAAAAAAAAAAAAAAAAAAAGAGAGCAGAUCUC UGUUGAAUAACCAACGUCAAUAUGCUGAAAAAAUCCAAAAAAAAU CUAAAAAAAUCUUUUAAAAAACCCCAAAAAAAUUUACAAAAAAGG AUCCACAGCAUAUUGACGCUGGGAAAGACCAGAGAUCUGCUCUCU | 89 |
| PLA51 | AAAAAAAAAAAAAAAAAAAAAAAAAAAAAAAAAAAAAAAAAAAAA AAAAAAAAAAAAAAAAAAAAAAAAAAAAAAAAAAAGAGAGCAGAU CUCUGUUGAAUAACCAACGUCAAUAUGCUGAAAAAAUCCAAAAAA AAUCUAAAAAAAUCUUUUAAAAAACCCCAAAAAAAUUUACAAAAA AGGAUCCACAGCAUAUUGACGCUGGGAAAGACCAGAGAUCUGCUC UCU | 90 |
| PLA170 | AAAAAAUCCAAAAAAAAUCUAAAAAAAUCUUUUAAAAAACCCCAA AAAAAUUUACAAAAAAGGAUCCAAGAUUAAUAAAAAAAAAAAAAA AAAAAAAAAAAAAAAAAAAAAAAAAAAAAAAAAAAGAGAGCAGAU CUCUGUUGAAUAACCAACGUCAAUAUGCUGAAAAAAAAAAAAAA AAAAAAAAAAAAAAAAAACAGCAUAUUGACGCUGGGAAAGACCAGA GAUCUGCUCUCU | 91 |
| PLA171 | AAAAAAUCCAAAAAAAAUCUAAAAAAAUCUUUUAAAAAACCCCAA AAAAAUUUACAAAAAAGGAUCCAAGAUUAAUAAAAAAAAAAAAAA AAAAAAAAAAAAAAAAAAAAAAAAAAAAAAAAAGAGAGCAGAUCU CUGUUGAAUAACCAACGUCAAUAUGCUGAAAAAAAAAAAACAGCA UAUUGACGCUGGGAAAGACCAGAGAUCUGCUCUCU | 92 |
| PLA178 | AAUAAAAAAAAUCCAAAAAAAAUCUAAAAAAAUCUUUAAAAA CCCCAAAAAAAUUUACAAAAAAGGAUCCAAGAUUAAUAAAAAAAA AAAAAAAAAAAAAAAAAAAAAAAAAAAAAAAAAAAAAAAAAGAGA GCAGAUCUCUGUUGAAUAACCAACGUCAAUAUGCUGAAAAAAAAA AAAAAAAAAAAAAAAAAAAAAAACAGCAUAUUGACGCUGGGAAAG ACCAGAGAUCUGCUCUCU | 93 |
| PLA179 | AAUAAAAAAAAAAUCCAAAAAAAAAUCUAAAAAAAAUCUUUUAAAAAA CCCCAAAAAAAAUUUACAAAAAAGGAUCCAAGAUUAAUAAAAAAAA AAAAAAAAAAAAAAAAAAAAAAAAAAAAAAAAAAAAAGAGAGC AGAUCUCUGUUGAAUAACCAACGUCAAUAUGCUGAAAAAAAAAAAA ACAGCAUAUUGACGCUGGGAAAGACCAGAGAUCUGCUCUCU | 94 |

| | | |
|---|---|---|
| (CS₁ and CS₂ sequences are lined) | | |

The constructs of { (A)ₘ-CS₁-(A)ₙ-CS₂-(A)ₒ}ₚ (Formula (I)) were produced by amplifying the poly(A) region by adding poly(A) to the CS₂ end, and protein expression efficiency was found to be increased compared to the A₁₀₀ control. These results demonstrate the technical potential to achieve additional superior efficacy through optimization because protein expression efficiency is increased even when sequence A is present after the CS₂ sequence without functional problems.

The constructs of [(A)ₘ-CS₁-{(A)ᵣ-(G/C/U) }ₓ-(A)_{y}-CS₂]ₚ (Formula (III)), [(A)ₘ-CS₁-{(A)ᵣ-(G/C/U)}ₓ-{(A)ₛ-(G/C/U) }_{x'}-CS₂]ₚ (Formula (IV)), [{ (A) ᵣ-(G/C/U) }ₓ-(A) _{y}-CS₁-(A)ₙ-CS₂] p (Formula (V)), [{(A)ᵣ-(G/C/U) }ₓ-(A)_{y}-CS₁-{ (A)ᵣ-(G/C/U)}_{x'}-(A)_{y'}-CS₂]ₚ (Formula (VI)), [{(A)ᵣ-(G/C/U)}ₓ-(A)_{y}-CS₁-{(A) ₛ-(G/C/U) }_{x'}-CS₂]ₚ (Formula (VII)), [{(A)ᵣ-(G/C/U) }ₓ-(A)_{y}-CS₁-{(A)ᵣ-(G/C/U) }_{x'}-{(A)ₛ-(G/C/U) }_{x'}-CS₂]ₚ (Formula (VIII)) were established by incorporating sequence combinations, designated Mixed tail or Non-A, which have functionally similar functions to poly(A), either individually or in both the proximal poly(A) and distal poly(A) regions. Efficacy testing showed that all constructs exhibited superior protein expression efficiency compared to the A₁₀₀ control. These results suggest that even non-A sequences, which have similar functions to typical poly(A) sequences, have the potential of increasing protein expression efficiency of the combination of CS₁ and CS₂ sequences.

The constructs {(N)_{q}-(A)ₘ-CS₁-(A)ₙ-CS₂}ₚ (Formula (II)) were produced by inserting a sequence, called ARS (adenine rich site), which has a similar function to poly(A) in the front of the proximal poly(A) region and were found to have an effect of enhancing protein expression efficiency, which suggests the technological potential to achieve superior efficacy.

### Example 6: Sequence and structure optimization of CS₁ and CS₂

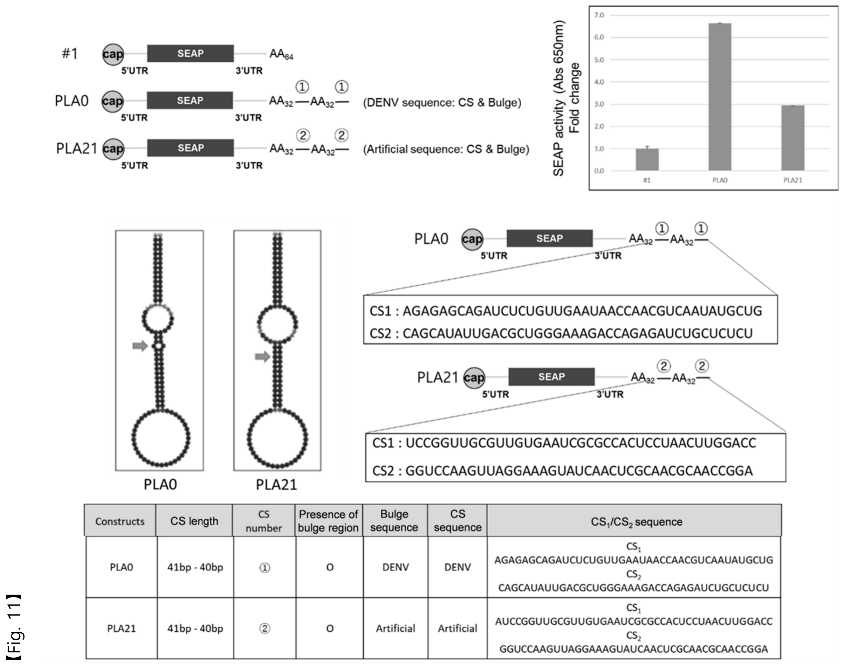

To verify the concept of enhancing RNA stability and protein expression efficiency by forming complementary binding sequences between CS₁ and CS₂ in the middle or end of poly(A), some of the sequences known to have no pathogenic effects among the DENV virus-derived sequences were modified, and applied and verified. Additionally, since complementary binding structure formation plays a crucial role in increasing protein production efficiency, the same effect may be expected even when replacing sequences other than those derived from the DENV virus with sequences capable of artificially inducing complementary binding structures. Therefore, the PLA21 construct was produced to verify this effect (FIG. 11).

The result showed that PLA21 increased protein production efficiency by twofold or more, compared to the A₆₄ control (#1) (FIG. 11). However, it was approximately twofold lower effect than that of the PLAO construct, which used a complementary binding sequence derived from the DENV virus. These differences were expected to be due to structural features specific to the complementary sequence derived from the DENV virus. To determine this, the structures of the PLA0 and PLA21 constructs were compared using RNAfold (http:/rna.tbi.univie.ac.at/cgi-bin/RNAWebSuite/RNAfold.cgi), a tool for predicting secondary structure formation. As a result, it was confirmed that the bulge region, which is specifically formed between the complementary binding structures of CS₁ and CS₂, differs between PLA0 and PLA21 (FIG. 11).

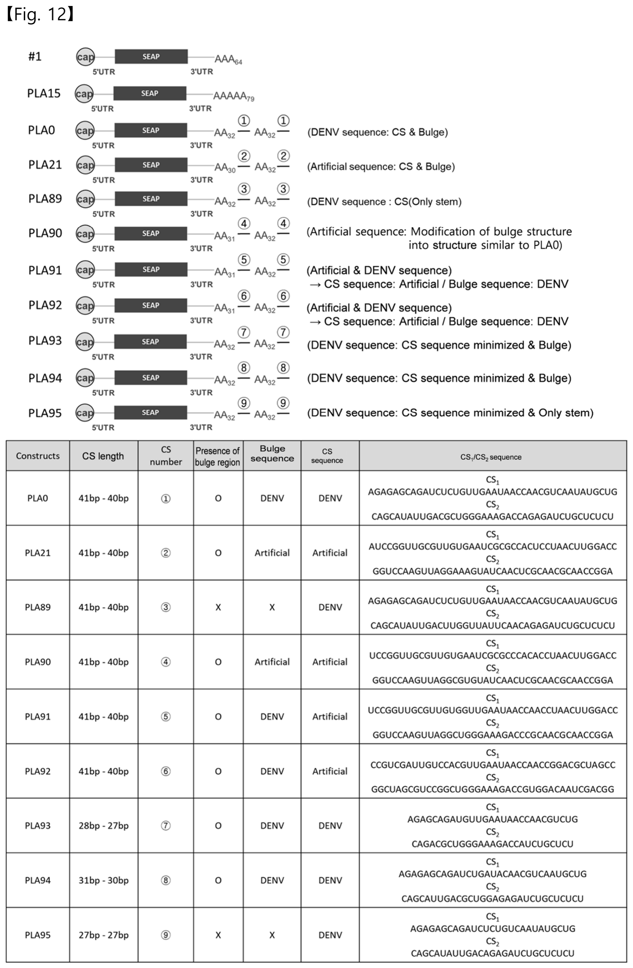

Recent studies have reported that double-strand structures of viral RNA longer than a predetermined length (>33 nt) may induce an innate immune response, but this may vary depending on the presence or absence of a bulge within the double-strand structure. In particular, this innate immune response is well known to inhibit the stability of the corresponding RNA and protein translation efficiency within cells. Therefore, the presence or absence of bulge formation and the structural morphology thereof would play a key role in regulating protein expression efficiency. To confirm this and identify the optimal complementary binding sequence between CS₁ and CS₂, constructs with various lengths and sequence combinations depending on bulge formation and complementary binding sequences were produced and tested (Table 5 and FIG. 12).

**[Table 5]**

| Poly(A) region sequences of constructs | | |
|---|---|---|
| **Constructs** | **Poly (A) region sequences comprising CS₁ and CS₂ sequences (5'->3')** | **SEQ ID NO.** |
| #1 | AAAAAAAAAAAAAAAAAAAAAAAAAAAAAAAAAAAAAAAAAAAAAAAAA AAAAAAAAAAAAAAAAAAAAA | 23 |
| PLA15 | AAAAAAAAAAAAAAAAAAAAAAAAAAAAAAAAAAAAAAAAAAAAAAAAAA AAAAAAAAAAAAAAAAAAAAAAAAAAAAAAAAAAAAAAA | 27 |
| PLAO | AAAAAAAAAAAAAAAAAAAAAAAAAAAAAAAAGAGAGCAGAUCUC UGUUGAAUAACCAACGUCAAUAUGCUGAAAAAAAAAAAAAAAAAA AAAAAAAAAAAAAACAGCAUAUUGACGCUGGGAAAGACCAGAGAU CUGCUCUCU | 24 |
| PLA21 | AAAAAAAAAAAAAAAAAAAAAAAAAAAAAAAUCCGGUUGCGUUGU GAAUCGCGCCACUCCUAACUUGGACCAAAAAAAAAAAAAAAAAA AAAAAAAAAAAAAAAGGUCCAAGUUAGGAAAGUAUCAACUCGCAACG CAACCGGA | 95 |
| PLA89 | AAAAAAAAAAAAAAAAAAAAAAAAAAAAAAAAAGAGAGCAGAUCUC UGUUGAAUAACCAACGUCAAUAUGCUGAAAAAAAAAAAAAAAAAA AAAAAAAAAAAAAAAACAGCAUAUUGACUUGGUUAUUCAACAGAGAU CUGCUCUCU | 96 |
| PLA90 | AAAAAAAAAAAAAAAAAAAAAAAAAAAAAAAAUCCGGUUGCGUUGU GAAUCGCGCCCACACCUAACUUGGACCAAAAAAAAAAAAAAAAA AAAAAAAAAAAAAAAGGUCCAAGUUAGGCGUGUAUCAACUCGCAAC GCAACCGGA | 97 |
| PLA91 | AAAAAAAAAAAAAAAAAAAAAAAAAAAAAAAAUCCGGUUGCGUUGU GGUUGAAUAACCAACCUAACUUGGACCAAAAAAAAAAAAAAAAA AAAAAAAAAAAAAAAAGGUCCAAGUUAGGCUGGGAAAGACCCGCAAC GCAACCGGA | 98 |
| PLA92 | AAAAAAAAAAAAAAAAAAAAAAAAAAAAAAAACCGUCGAUUGUCCA CGUUGAAUAACCAACCGGACGCUAGCCAAAAAAAAAAAAAAAAAAA AAAAAAAAAAAAAAAGGCUAGCGUCCGGCUGGGAAAGACCGUGGAC AAUCGACGG | 99 |
| PLA93 | AAAAAAAAAAAAAAAAAAAAAAAAAAAAAAAAGAGCAGAUGUUGA AUAACCAACGUCUGAAAAAAAAAAAAAAAAAAAAAAAAAAAAAAA ACAGACGCUGGGAAAGACCAUCUGCUCU | 100 |
| PLA94 | AAAAAAAAAAAAAAAAAAAAAAAAAAAAAAAAGAGAGCAGAUCUG AUACAACGUCAAUGCUGAAAAAAAAAAAAAAAAAAAAAAAAAAAA AAAACAGCAUUGACGCUGGAGAGAUCUGCUCUCU | 101 |
| PLA95 | AAAAAAAAAAAAAAAAAAAAAAAAAAAAAAAGAGAGCAGAUCUC UGUCAAUAUGCUGAAAAAAAAAAAAAAAAAAAAAAAAAAAAAAAA CAGCAUAUUGACAGAGAUCUGCUCUCU | 102 |

| | | |
|---|---|---|
| (CS₁ and CS₂ sequences are lined) | | |

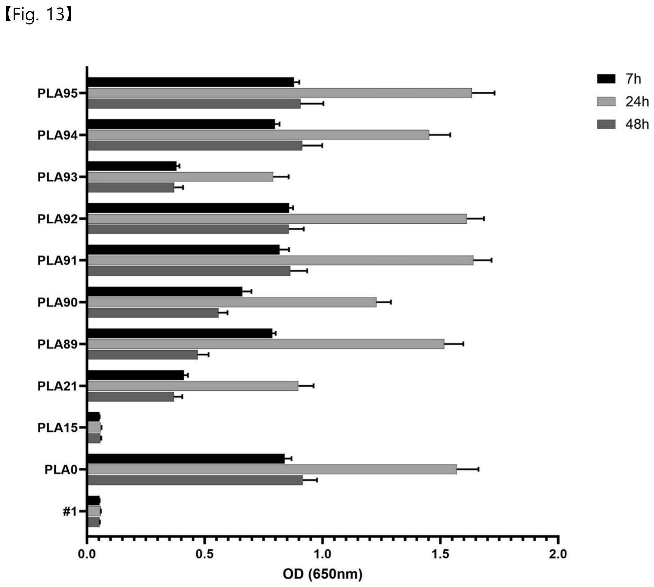

As a result, it was confirmed that the complementary sequence was present at 30% to 100%, and that the protein expression efficiency was significantly increased when the bulge structure was present in the DENV-derived complementary sequence (FIG. 13).

### Example 7: Verification of protein expression efficiency of nucleic acid constructs in which GOI of candidates with increased protein expression efficiency is changed to Gaussia luciferase

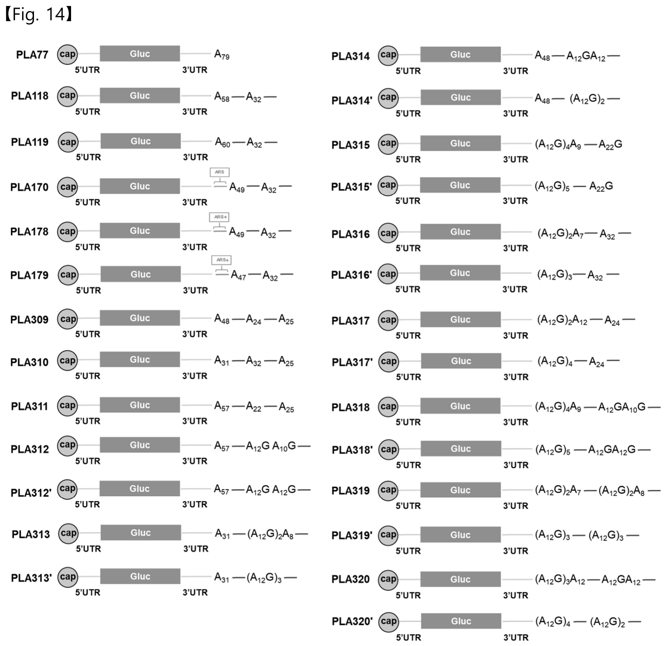

The SEAP gene sequence was used as the GOI (Gene of Interest) for candidate screening in Examples 4 to 6. To determine whether or not the effect of secondary structure formation through complementary binding of CS on protein expression efficiency was also observed in other GOIs, tests were conducted by replacing the SEAP-encoding gene sequence with the Gaussia luciferase gene sequence. The tests were performed using the candidates top-ranked in Examples 4 and 6 (FIG. 14).

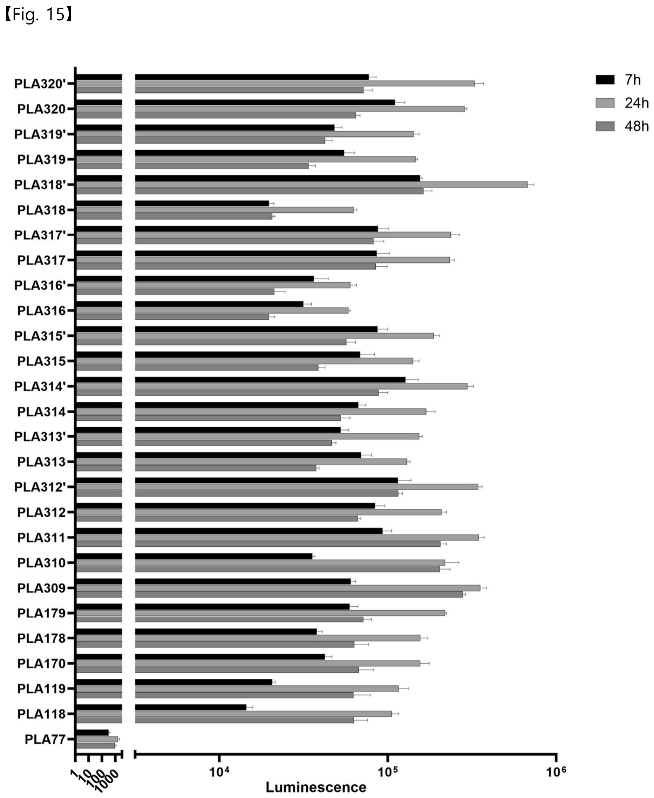

The result showed that the constructs of Formula (I) to Formula (VIII) induced significantly increased protein expression in GOIs other than SEAP (FIG. 15).

### Example 8: Verification of protein expression efficiency of nucleic acid constructs comprising various UTRs

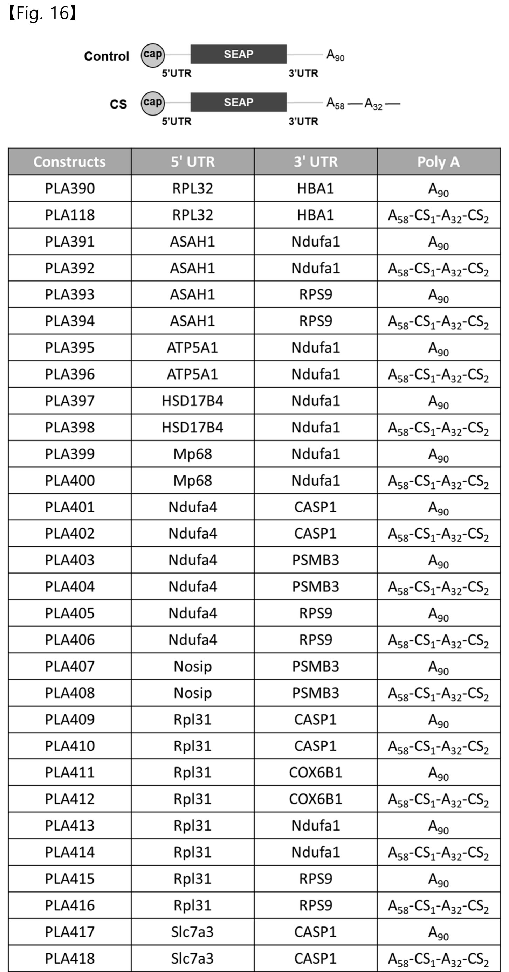

The 5'-UTR and the 3'-UTR used in the screening tests of Examples 4 to 6 were RPL32 and HBA1, respectively. To determine whether or not secondary structure formation through complementary binding of CS also enhances protein expression efficiency in other UTRs, protein expression efficiencies were compared after applying 20 different UTRs (Table 6, FIG. 16).

**[Table 6]**

| 5'-UTR, 3'-UTR, and poly(A) region sequences of the structure (5'->3') | | | |
|---|---|---|---|
| **Constructs** | **5'-UTR** | **3'-UTR** | **Poly (A) region sequence** |
| PLA390 | GGCGCUGCCUA CGGAGGUGGCA GCCAUCUCCUU CUCGGCAUC (SEQ ID NO. 19) | GCCCGAUGGCCUCCC AACGGGCCCUCCUCCC CUCCUUGCACCG (SEQ ID NO. 20) | AAAAAAAAAAAAAAAAA AAAAAAAAAAAAAAAAA AAAAAAAAAAAAAAAAA AAAAAAAAAAAAAAAAA AAAAAAAAAAAAAAAAA AAAAAAAAAA (SEQ ID NO. 116) |
| PLA391 | GGCUCUUCUUU GCCUCUGCUGG AGUCCGGGGAG UGGCGUUGGCU GCUAGAGCG (SEQ ID NO. | GGAAGCAUUUUCCUGA UUGAUGAAAAAAAUAA CUCAGUUAUGGCCAUC UACCCCUGCUAGAAGG UUACAGUGUAUUAUGU AGCAUGCAAUGUGUUA | AAAAAAAAAAAAAAAAAA AAAAAAAAAAAAAAAAAA AAAAAAAAAAAAAAAAAA AAAAAAAAAAAAAAAAAA AAAAAAAAAAAAAAAAAA AAAAAAAAAAA |
| | 103) | UGUAGUGCUUAAUAAA AAUAAAAUG (SEQ ID NO. 111) | (SEQ ID NO. 116) |
| PLA392 | GGCUCUUCUUU GCCUCUGCUGG AGUCCGGGGAG UGGCGUUGGCU GCUAGAGCG (SEQ ID NO. 103) | GGAAGCAUUUUCCUGA UUGAUGAAAAAAAUAA CUCAGUUAUGGCCAUC UACCCCUGCUAGAAGG UUACAGUGUAUUAUGU AGCAUGCAAUGUGUUA UGUAGUGCUUAAUAAA AAUAAAAUG (SEQ ID NO. 111) | AAAAAAAAAAAAAAAA AAAAAAAAAAAAAAAA AAAAAAAAAAAAAAAA AAAAAAAAAAGAGAGC AGAUCUCUGUUGAAUA ACCAACGUCAAUAUGC UGAAAAAAAAAAAAAA AAAAAAAAAAAAAAAA AACAGCAUAUUGACGC UGGGAAAGACCAGAGA UCUGCUCUCU (SEQ ID NO. 51) |
| PLA393 | GGCUCUUCUUU GCCUCUGCUGG AGUCCGGGGAG UGGCGUUGGCU GCUAGAGCG (SEQ ID NO. 103) | GUCCACCUGUCCCUCC UGGGCUGCUGGAUUGU CUCGUUUUCCUGCCAA AUAAACAGGAUCAGCG CUUUAC (SEQ ID NO. 112) | AAAAAAAAAAAAAAAAA AAAAAAAAAAAAAAAAA AAAAAAAAAAAAAAAAA AAAAAAAAAAAAAAAAA AAAAAAAAAAAAAAAAA AAAAAAAAAA (SEQ ID NO. 116) |
| PLA394 | GGCUCUUCUUU GCCUCUGCUGG AGUCCGGGGAG UGGCGUUGGCU GCUAGAGCG (SEQ ID NO. 103) | GUCCACCUGUCCCUCC UGGGCUGCUGGAUUGU CUCGUUUUCCUGCCAA AUAAACAGGAUCAGCG CUUUAC (SEQ ID NO. 112) | AAAAAAAAAAAAAAAA AAAAAAAAAAAAAAAA AAAAAAAAAAAAAAAA AAAAAAAAAAGAGAGC AGAUCUCUGUUGAAUA ACCAACGUCAAUAUGC UGAAAAAAAAAAAAAA AAAAAAAAAAAAAAAA AACAGCAUAUUGACGC UGGGAAAGACCAGAGA UCUGCUCUCU (SEQ ID NO. 51) |
| PLA395 | CCUUCUUUGCG GCUCGGCCAUU UUGUCCCAGUC AGUCCGGAGGC UGCGGCUGCAG AAGUACCGCCU GCGGAGUAACU GCAAAG (SEQ ID NO. 104) | GGAAGCAUUUUCCUGA UUGAUGAAAAAAAUAA CUCAGUUAUGGCCAUC UACCCCUGCUAGAAGG UUACAGUGUAUUAUGU AGCAUGCAAUGUGUUA UGUAGUGCUUAAUAAA AAUAAAAUG (SEQ ID NO. 111) | AAAAAAAAAAAAAAAA AAAAAAAAAAAAAAAA AAAAAAAAAAAAAAAA AAAAAAAAAAAAAAAA AAAAAAAAAAAAAAAA AAAAAAAAAA (SEQ ID NO. 116) |
| PLA396 | CCUUCUUUGCG GCUCGGCCAUU UUGUCCCAGUC AGUCCGGAGGC | GGAAGCAUUUUCCUGA UUGAUGAAAAAAAUAA CUCAGUUAUGGCCAUC UACCCCUGCUAGAAGG | AAAAAAAAAAAAAAAA AAAAAAAAAAAAAAAA AAAAAAAAAAAAAAAA AAAAAAAAAAGAGAGC |
| | UGCGGCUGCAG AAGUACCGCCU GCGGAGUAACU GCAAAG (SEQ ID NO. 104) | UUACAGUGUAUUAUGU AGCAUGCAAUGUGUUA UGUAGUGCUUAAUAAA AAUAAAAUG (SEQ ID NO. 111) | AGAUCUCUGUUGAAUA ACCAACGUCAAUAUGC UGAAAAAAAAAAAAAA AAAAAAAAAAAAAAAA AACAGCAUAUUGACGC UGGGAAAGACCAGAGA UCUGCUCUCU (SEQ ID NO. 51) |
| PLA397 | CCGCCUCCUCC UGUCCCGCAGU CGGCGUCCAGC GGCUCUGCUUG UUCGUGUGUGU GUCGUUGCAGG CCUUAUUC (SEQ ID NO. 105) | GGAAGCAUUUUCCUGA UUGAUGAAAAAAAUAA CUCAGUUAUGGCCAUC UACCCCUGCUAGAAGG UUACAGUGUAUUAUGU AGCAUGCAAUGUGUUA UGUAGUGCUUAAUAAA AAUAAAAUG (SEQ ID NO. 111) | AAAAAAAAAAAAAAAA AAAAAAAAAAAAAAAA AAAAAAAAAAAAAAAA AAAAAAAAAAAAAAAA AAAAAAAAAAAAAAAA AAAAAAAAAA (SEQ ID NO. 116) |
| PLA398 | CCGCCUCCUCC UGUCCCGCAGU CGGCGUCCAGC GGCUCUGCUUG UUCGUGUGUGU GUCGUUGCAGG CCUUAUUC (SEQ ID NO. 105) | GGAAGCAUUUUCCUGA UUGAUGAAAAAAAUAA CUCAGUUAUGGCCAUC UACCCCUGCUAGAAGG UUACAGUGUAUUAUGU AGCAUGCAAUGUGUUA UGUAGUGCUUAAUAAA AAUAAAAUG (SEQ ID NO. 111) | AAAAAAAAAAAAAAAAA AAAAAAAAAAAAAAAAA AAAAAAAAAAAAAAAAA AAAAAAAAAAAGAGAGC AGAUCUCUGUUGAAUA ACCAACGUCAAUAUGC UGAAAAAAAAAAAAAAA AAAAAAAAAAAAAAAAA CAGCAUAUUGACGC UGGGAAAGACCAGAGA UCUGCUCUCU (SEQ ID NO. 51) |
| PLA399 | GACCUUUCCGA GUUGGCUGCAG AUUUGUGGUGC GUUCUGAGCCG UCUGUCCUGCG CCAAG (SEQ ID NO. 106) | GGAAGCAUUUUCCUGA UUGAUGAAAAAAAUAA CUCAGUUAUGGCCAUC UACCCCUGCUAGAAGG UUACAGUGUAUUAUGU AGCAUGCAAUGUGUUA UGUAGUGCUUAAUAAA AAUAAAAUG (SEQ ID NO. 111) | AAAAAAAAAAAAAAAAAA AAAAAAAAAAAAAAAAAA AAAAAAAAAAAAAAAAAA AAAAAAAAAAAAAAAAAA AAAAAAAAAAAAAAAAAA AAAAAAAAAA (SEQ ID NO. 116) |
| PLA400 | GACCUUUCCGA GUUGGCUGCAG AUUUGUGGUGC GUUCUGAGCCG UCUGUCCUGCG CCAAG (SEQ ID NO. 106) | GGAAGCAUUUUCCUGA UUGAUGAAAAAAAUAA CUCAGUUAUGGCCAUC UACCCCUGCUAGAAGG UUACAGUGUAUUAUGU AGCAUGCAAUGUGUUA UGUAGUGCUUAAUAAA AAUAAAAUG (SEQ ID NO. 111) | AAAAAAAAAAAAAAAA AAAAAAAAAAAAAAAA AAAAAAAAAAAAAAAA AAAAAAAAAAGAGAGC AGAUCUCUGUUGAAUA ACCAACGUCAAUAUGC UGAAAAAAAAAAAAAA AAAAAAAAAAAAAAAA AACAGCAUAUUGACGC UGGGAAAGACCAGAGA |
| | | | UCUGCUCUCU (SEQ ID NO. 51) |
| PLA401 | GGAAGUCCGUA GUGUCUCAUUG CAGAUAAUUUU UAGCUUAGGGC CUGGUGGCUAG GUCGGUUCUCU CCUUUCCAGUC GGAGACCUCUG CCGCAAAC (SEQ ID NO. 107) | AAUAAGGAAACUGUAU GAAUGUCUGUGGGCAG GAAGUGAAGAGAUCCU UCUGUAAAGGUUUUUG GAAUUAUGUCUGCUGA AUAAUAAACUUUUUUG AAAUAAUAAAUCUGGU AGAAAAAUGAAAACUU GUCCUCAUUUUUCUCC CACACUGAAGAAACAG GGACUGGAACUUAGAG UGACUAAGGAAUUU (SEQ ID NO. 113) | AAAAAAAAAAAAAAAAA AAAAAAAAAAAAAAAAA AAAAAAAAAAAAAAAAA AAAAAAAAAAAAAAAAA AAAAAAAAAAAAAAAAA AAAAAAAAAA (SEQ ID NO. 116) |
| PLA402 | GGAAGUCCGUA GUGUCUCAUUG CAGAUAAUUUU UAGCUUAGGGC CUGGUGGCUAG GUCGGUUCUCU CCUUUCCAGUC GGAGACCUCUG CCGCAAAC (SEQ ID NO. 107) | AAUAAGGAAACUGUAU GAAUGUCUGUGGGCAG GAAGUGAAGAGAUCCU UCUGUAAAGGUUUUUG GAAUUAUGUCUGCUGA AUAAUAAACUUUUUUG AAAUAAUAAAUCUGGU AGAAAAAUGAAAACUU GUCCUCAUUUUUCUCC CACACUGAAGAAACAG GGACUGGAACUUAGAG UGACUAAGGAAUUU (SEQ ID NO. 113) | AAAAAAAAAAAAAAAAA AAAAAAAAAAAAAAAAA AAAAAAAAAAAAAAAAA AAAAAAAAAAAGAGAGC AGAUCUCUGUUGAAUA ACCAACGUCAAUAUGC UGAAAAAAAAAAAAAAA AAAAAAAAAAAAAAAAA CAGCAUAUUGACGC UGGGAAAGACCAGAGA UCUGCUCUCU (SEQ ID NO. 51) |
| PLA403 | GGAAGUCCGUA GUGUCUCAUUG CAGAUAAUUUU UAGCUUAGGGC CUGGUGGCUAG GUCGGUUCUCU CCUUUCCAGUC GGAGACCUCUG CCGCAAAC (SEQ ID NO. 107) | CCCUGUUCCCAGAGCC CACUUUUUUUCUUUU UUUGAAUAAAAUAGC CUGUCUUUC (SEQ ID NO. 114) | AAAAAAAAAAAAAAAAAA AAAAAAAAAAAAAAAAAA AAAAAAAAAAAAAAAAAA AAAAAAAAAAAAAAAAAA AAAAAAAAAAAAAAAAAA AAAAAAAAAA (SEQ ID NO. 116) |
| PLA404 | GGAAGUCCGUA GUGUCUCAUUG CAGAUAAUUUU UAGCUUAGGGC CUGGUGGCUAG GUCGGUUCUCU CCUUUCCAGUC GGAGACCUCUG CCGCAAAC | CCCUGUUCCCAGAGCC CACUUUUUUUUCUUUU UUUGAAAUAAAAUAGC CUGUCUUUC (SEQ ID NO. 114) | AAAAAAAAAAAAAAAAA AAAAAAAAAAAAAAAAA AAAAAAAAAAAAAAAAA AAAAAAAAAAGAGAGC AGAUCUCUGUUGAAUA ACCAACGUCAAUAUGC UGAAAAAAAAAAAAAA AAAAAAAAAAAAAAAAA ACAGCAUAUUGACGC |
| | (SEQ ID NO. 107) | | UGGGAAAGACCAGAGA UCUGCUCUCU (SEQ ID NO. 51) |
| PLA405 | GGAAGUCCGUA GUGUCUCAUUG CAGAUAAUUUU UAGCUUAGGGC CUGGUGGCUAG GUCGGUUCUCU CCUUUCCAGUC GGAGACCUCUG CCGCAAAC (SEQ ID NO. 107) | GUCCACCUGUCCCUCC UGGGCUGCUGGAUUGU CUCGUUUUCCUGCCAA AUAAACAGGAUCAGCG CUUUAC (SEQ ID NO. 112) | AAAAAAAAAAAAAAAAA AAAAAAAAAAAAAAAAA AAAAAAAAAAAAAAAAA AAAAAAAAAAAAAAAAA AAAAAAAAAAAAAAAAA AAAAAAAAAA (SEQ ID NO. 116) |
| PLA406 | GGAAGUCCGUA GUGUCUCAUUG CAGAUAAUUUU UAGCUUAGGGC CUGGUGGCUAG GUCGGUUCUCU CCUUUCCAGUC GGAGACCUCUG CCGCAAAC (SEQ ID NO. 107) | GUCCACCUGUCCCUCC UGGGCUGCUGGAUUGU CUCGUUUUCCUGCCAA AUAAACAGGAUCAGCG CUUUAC (SEQ ID NO. 112) | AAAAAAAAAAAAAAAAA AAAAAAAAAAAAAAAAA AAAAAAAAAAAAAAAAA AAAAAAAAAAGAGAGC AGAUCUCUGUUGAAUA ACCAACGUCAAUAUGC UGAAAAAAAAAAAAAA AAAAAAAAAAAAAAAAA ACAGCAUAUUGACGC UGGGAAAGACCAGAGA UCUGCUCUCU (SEQ ID NO. 51) |
| PLA407 | ACAGUUGAAGA AGCGACCGAGG GACUGGGAGUC GUUAGUGAGG (SEQ ID NO. 108) | CCCUGUUCCCAGAGCC CACUUUUUUUCUUUU UUUGAAUAAAAUAGC CUGUCUUUC (SEQ ID NO. 114) | AAAAAAAAAAAAAAAAA AAAAAAAAAAAAAAAAA AAAAAAAAAAAAAAAAA AAAAAAAAAAAAAAAAA AAAAAAAAAAAAAAAAA AAAAAAAAAA (SEQ ID NO. 116) |
| PLA408 | ACAGUUGAAGA AGCGACCGAGG GACUGGGAGUC GUUAGUGAGG (SEQ ID NO. 108) | CCCUGUUCCCAGAGCC CACUUUUUUUUCUUUU UUUGAAAUAAAAUAGC CUGUCUUUC (SEQ ID NO. 114) | AAAAAAAAAAAAAAAA AAAAAAAAAAAAAAAA AAAAAAAAAAAAAAAA AAAAAAAAAAGAGAGC AGAUCUCUGUUGAAUA ACCAACGUCAAUAUGC UGAAAAAAAAAAAAAA AAAAAAAAAAAAAAAA AACAGCAUAUUGACGC UGGGAAAGACCAGAGA UCUGCUCUCU (SEQ ID NO. 51) |
| PLA409 | CUUCCUUUCCA ACUUGGACGCU GCAGA | AAUAAGGAAACUGUAU GAAUGUCUGUGGGCAG GAAGUGAAGAGAUCCU | AAAAAAAAAAAAAAAAAA AAAAAAAAAAAAAAAAAA AAAAAAAAAAAAAAAAAA |
| | (SEQ ID NO. 109) | UCUGUAAAGGUUUUUG GAAUUAUGUCUGCUGA AUAAUAAACUUUUUUG AAAUAAUAAAUCUGGU AGAAAAAUGAAAACUU GUCCUCAUUUUUCUCC CACACUGAAGAAACAG GGACUGGAACUUAGAG UGACUAAGGAAUUU (SEQ ID NO. 113) | AAAAAAAAAAAAAAAAA AAAAAAAAAAAAAAAAA AAAAAAAAAA (SEQ ID NO. 116) |
| PLA410 | CUUCCUUUCCA ACUUGGACGCU GCAGA (SEQ ID NO. 109) | AAUAAGGAAACUGUAU GAAUGUCUGUGGGCAG GAAGUGAAGAGAUCCU UCUGUAAAGGUUUUUG GAAUUAUGUCUGCUGA AUAAUAAACUUUUUUG AAAUAAUAAAUCUGGU AGAAAAAUGAAAACUU GUCCUCAUUUUUCUCC CACACUGAAGAAACAG GGACUGGAACUUAGAG UGACUAAGGAAUUU (SEQ ID NO. 113) | AAAAAAAAAAAAAAAA AAAAAAAAAAAAAAAA AAAAAAAAAAAAAAAA AAAAAAAAAAGAGAGC AGAUCUCUGUUGAAUA ACCAACGUCAAUAUGC UGAAAAAAAAAAAAAA AAAAAAAAAAAAAAAA AACAGCAUAUUGACGC UGGGAAAGACCAGAGA UCUGCUCUCU (SEQ ID NO. 51) |
| PLA411 | CUUCCUUUCCA ACUUGGACGCU GCAGA (SEQ ID NO. 109) | ACUGGCUGCAUCUCCC UUUCCUCUGUCCUCCA UCCUUCUCCCAGGAUG GUGAAGGGGGACCUGG UACCCAGUGAUCCCCA CCCCAGGAUCCUAAAU CAUGACUUACCUGCUA AUAAAAACUCAUUGGA AAAGUG (SEQ ID NO. 115) | AAAAAAAAAAAAAAAAA AAAAAAAAAAAAAAAAA AAAAAAAAAAAAAAAAA AAAAAAAAAAAAAAAAA AAAAAAAAAAAAAAAAA AAAAAAAAAA (SEQ ID NO. 116) |
| PLA412 | CUUCCUUUCCA ACUUGGACGCU GCAGA (SEQ ID NO. 109) | ACUGGCUGCAUCUCCC UUUCCUCUGUCCUCCA UCCUUCUCCCAGGAUG GUGAAGGGGGACCUGG UACCCAGUGAUCCCCA CCCCAGGAUCCUAAAU CAUGACUUACCUGCUA AUAAAAACUCAUUGGA AAAGUG (SEQ ID NO. 115) | AAAAAAAAAAAAAAAAA AAAAAAAAAAAAAAAAA AAAAAAAAAAAAAAAAA AAAAAAAAAAGAGAGC AGAUCUCUGUUGAAUA ACCAACGUCAAUAUGC UGAAAAAAAAAAAAAA AAAAAAAAAAAAAAAAA ACAGCAUAUUGACGC UGGGAAAGACCAGAGA UCUGCUCUCU (SEQ ID NO. 51) |
| PLA413 | CUUCCUUUCCA ACUUGGACGCU GCAGA | GGAAGCAUUUUCCUGA UUGAUGAAAAAAAUAA CUCAGUUAUGGCCAUC | AAAAAAAAAAAAAAAAAA AAAAAAAAAAAAAAAAAA AAAAAAAAAAAAAAAAAA |
| | (SEQ ID NO. 109) | UACCCCUGCUAGAAGG UUACAGUGUAUUAUGU AGCAUGCAAUGUGUUA UGUAGUGCUUAAUAAA AAUAAAAUG (SEQ ID NO. 111) | AAAAAAAAAAAAAAAAA AAAAAAAAAAAAAAAAA AAAAAAAAAA (SEQ ID NO. 116) |
| PLA414 | CUUCCUUUCCA ACUUGGACGCU GCAGA (SEQ ID NO. 109) | GGAAGCAUUUUCCUGA UUGAUGAAAAAAAUAA CUCAGUUAUGGCCAUC UACCCCUGCUAGAAGG UUACAGUGUAUUAUGU AGCAUGCAAUGUGUUA UGUAGUGCUUAAUAAA AAUAAAAUG (SEQ ID NO. 111) | AAAAAAAAAAAAAAAAA AAAAAAAAAAAAAAAAA AAAAAAAAAAAAAAAAA AAAAAAAAAAGAGAGC AGAUCUCUGUUGAAUA ACCAACGUCAAUAUGC UGAAAAAAAAAAAAAA AAAAAAAAAAAAAAAAA ACAGCAUAUUGACGC UGGGAAAGACCAGAGA UCUGCUCUCU (SEQ ID NO. 51) |
| PLA415 | CUUCCUUUCCA ACUUGGACGCU GCAGA (SEQ ID NO. 109) | GUCCACCUGUCCCUCC UGGGCUGCUGGAUUGU CUCGUUUUCCUGCCAA AUAAACAGGAUCAGCG CUUUAC (SEQ ID NO. 112) | AAAAAAAAAAAAAAAA AAAAAAAAAAAAAAAA AAAAAAAAAAAAAAAA AAAAAAAAAAAAAAAA AAAAAAAAAAAAAAAA AAAAAAAAAA (SEQ ID NO. 116) |
| PLA416 | CUUCCUUUCCA ACUUGGACGCU GCAGA (SEQ ID NO. 109) | GUCCACCUGUCCCUCC UGGGCUGCUGGAUUGU CUCGUUUUCCUGCCAA AUAAACAGGAUCAGCG CUUUAC (SEQ ID NO. 112) | AAAAAAAAAAAAAAAAA AAAAAAAAAAAAAAAAA AAAAAAAAAAAAAAAAA AAAAAAAAAAAGAGAGC AGAUCUCUGUUGAAUA ACCAACGUCAAUAUGC UGAAAAAAAAAAAAAA AAAAAAAAAAAAAAAAA ACAGCAUAUUGACGC UGGGAAAGACCAGAGA UCUGCUCUCU (SEQ ID NO. 51) |
| PLA417 | AAGCCGCAGCU UUGAAGCCUGA GCGGCCGAACU CGGCAGCUCCA ACCCAACUCGG CUUAACUCCGC CUCACCGAGCC CAGUCCAAGAC UCUGUGCUCCC UAGGUUUGCAA CAGCUCUCUGG | AAUAAGGAAACUGUAU GAAUGUCUGUGGGCAG GAAGUGAAGAGAUCCU UCUGUAAAGGUUUUUG GAAUUAUGUCUGCUGA AUAAUAAACUUUUUUG AAAUAAUAAAUCUGGU AGAAAAAUGAAAACUU GUCCUCAUUUUUCUCC CACACUGAAGAAACAG GGACUGGAACUUAGAG | AAAAAAAAAAAAAAAAAA AAAAAAAAAAAAAAAAAA AAAAAAAAAAAAAAAAAA AAAAAAAAAAAAAAAAAA AAAAAAAAAAAAAAAAAA AAAAAAAAAAA (SEQ ID NO. 116) |
| | (SEQ ID NO. 110) | UGACUAAGGAAUUU (SEQ ID NO. 113) | |
| PLA418 | AAGCCGCAGCU UUGAAGCCUGA GCGGCCGAACU CGGCAGCUCCA ACCCAACUCGG CUUAACUCCGC CUCACCGAGCC CAGUCCAAGAC UCUGUGCUCCC UAGGUUUGCAA CAGCUCUCUGG (SEQ ID NO. 110) | AAUAAGGAAACUGUAU GAAUGUCUGUGGGCAG GAAGUGAAGAGAUCCU UCUGUAAAGGUUUUUG GAAUUAUGUCUGCUGA AUAAUAAACUUUUUUG AAAUAAUAAAUCUGGU AGAAAAAUGAAAACUU GUCCUCAUUUUUCUCC CACACUGAAGAAACAG GGACUGGAACUUAGAG UGACUAAGGAAUUU (SEQ ID NO. 113) | AAAAAAAAAAAAAAAA AAAAAAAAAAAAAAAA AAAAAAAAAAAAAAAA AAAAAAAAAAGAGAGC AGAUCUCUGUUGAAUA ACCAACGUCAAUAUGC UGAAAAAAAAAAAAAA AAAAAAAAAAAAAAAA AACAGCAUAUUGACGC UGGGAAAGACCAGAGA UCUGCUCUCU (SEQ ID NO. 51) |

| | | | |
|---|---|---|---|
| (CS₁ and CS₂ sequences are lined) | | | |

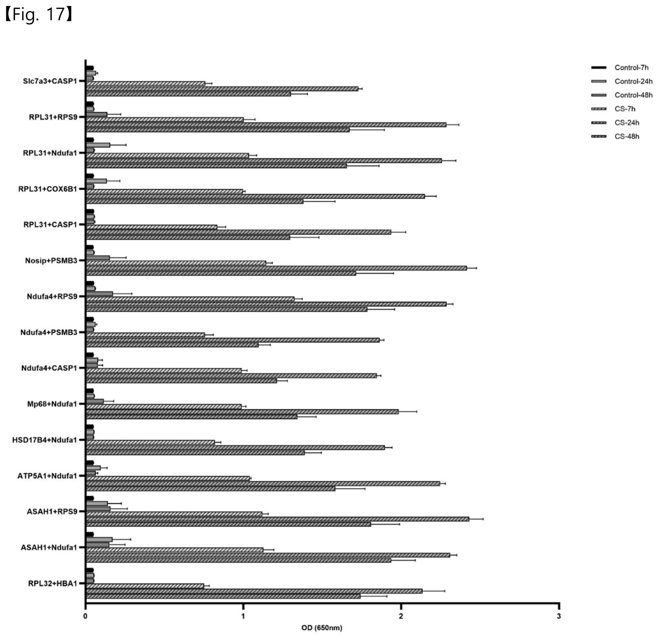

As a result, it was confirmed that when CS₁ and CS₂, which are the constructs according to the present invention, were applied to all UTRs, protein expression efficiency increased by more than twofold compared to the A₉₀ control (FIG. 17).

### Example 9: Verification of stability of poly(A) region during pDNA amplification in finally selected constructs

Most mRNA synthesis processes for therapeutic applications involve linearizing pDNA containing an uninterrupted A-T base pair region encoding a poly(A) tail, followed by *in vitro* transcription (IVT) using this as a template. pDNA is obtained from *Escherichia coli (E. coli)* cultures lacking recombinase. However, even in E. *coli* lacking recombinase, homopolymer regions, where identical sequences such as the poly(A) tail regions repeat for a predetermined length or more, are highly unstable and are often cleaved during replication and cell division in *E. coli* cultures. This phenomenon increases proportionally with increasing homopolymer length. It was expected that fractionation of the homopolymer region within the template pDNA due to the introduction of CS₁ may cause a relatively shorter continuous A sequence, ultimately enhancing the stability of the poly(A) region during pDNA replication in *E. coli.*

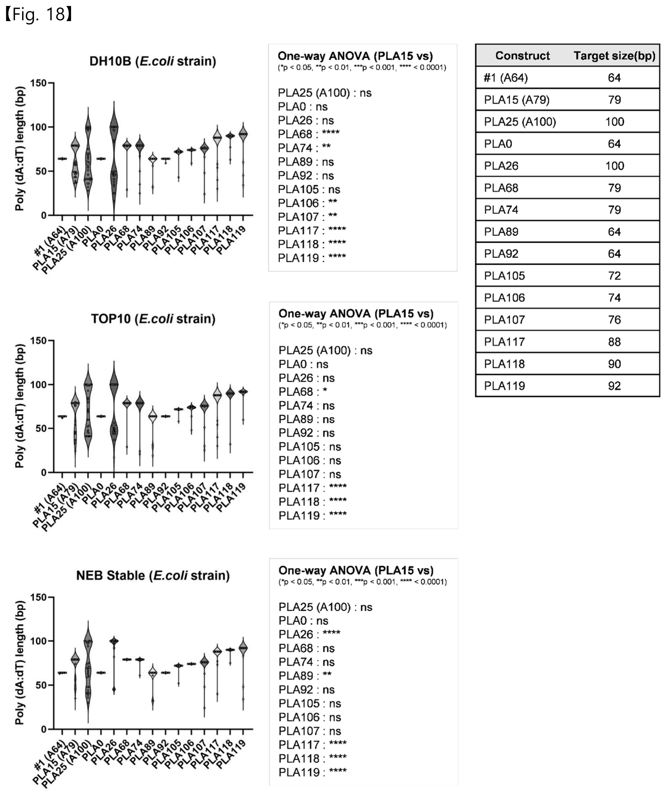

To confirm this, stability tests for the poly(A) region during pDNA replication were conducted in three different strains of *E. coli* (DH10B, TOP10, and NEB^{®}Stable). The results showed that pDNAs containing continuous A₇₉ or A₁₀₀ have a truncated portion of the A sequence in the poly(A) tail and thus reduce the stability of the poly(A) region. On the other hand, this phenomenon was reduced in constructs comprising CS₁ and CS₂, which indicates improved stability of the poly(A) region in pDNA (FIG. 18).

### Example 10: Inhibition of double-stranded RNA (dsRNA) production during in-vitro transcription (IVT)

During *in vitro* transcription (IVT), double-stranded RNA (dsRNA) is produced as a byproduct of the reaction. Upon entry into cells, the produced double-stranded RNA (dsRNA) acts as an agonist for toll-like receptor 3 (TLR3) and melanoma differentiation-associated protein 5 (MDA5), inducing cytokine release and exhibiting immunogenicity. Double-stranded RNA-induced immunogenicity induces cytotoxicity. Furthermore, mechanisms in which RNA introduced into the body is recognized as viral RNA and degraded due to immunogenicity reduce RNA translation efficiency. Therefore, controlling immunogenicity, particularly reducing the production of double-stranded RNA, is essential for enhancing the efficacy of vaccines or gene therapy medicaments.

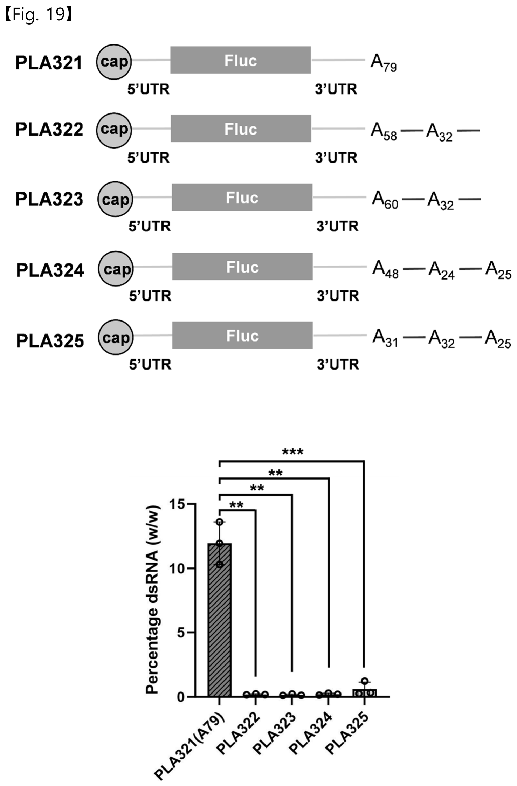

In this example, a test was conducted to determine whether or not the complementary binding of the construct comprising CS₁ and CS₂ resulted in recognition of the corresponding region as double-stranded RNA. The amount of dsRNA was determined by enzyme-linked immunosorbent assay (ELISA) using an anti-dsRNA J2 antibody. The GOI of the top-ranked candidate in Examples 4 and 6 was replaced with the FLuc gene sequence (FIG. 19).

As a result, when IVT was performed using the same method, the complementary binding between CS₁ and CS₂ was not recognized as dsRNA by the anti-dsRNA J2 antibody and, when CS was applied, the amount of double-stranded RNA produced after the IVT reaction was significantly reduced compared to the control (FIG. 19).

### Example 11: Verification of immunogenicity of finally selected construct

RNA induces inflammation through multiple pathways, particularly through the innate immune system, which has numerous RNA-detecting sensors. An RNA-induced innate immune response may reduce intracellular RNA translation, or induce systemic/local toxicity and inflammatory responses, which may impact the anticipated efficacy and safety of pharmaceuticals.

In order to assess the degree of innate immune response induced by the construct according to the present invention, cytokine expression patterns depending on introduction of RNA into monocytes and macrophages were analyzed.

The mRNA containing the final selected poly(A) tail construct from Example 10 was delivered to THP-1 cells, a human cell line derived from acute myeloid leukemia, and macrophages derived from THP-1 cells differentiated with phorbol 12-myristate 13-acetate (PMA) using MessengerMAX^{™}. The concentrations of Type I interferon, inflammatory cytokines, and chemokines secreted as a result of the innate immune response were measured in the culture medium 6 and 24 hours after transfection.

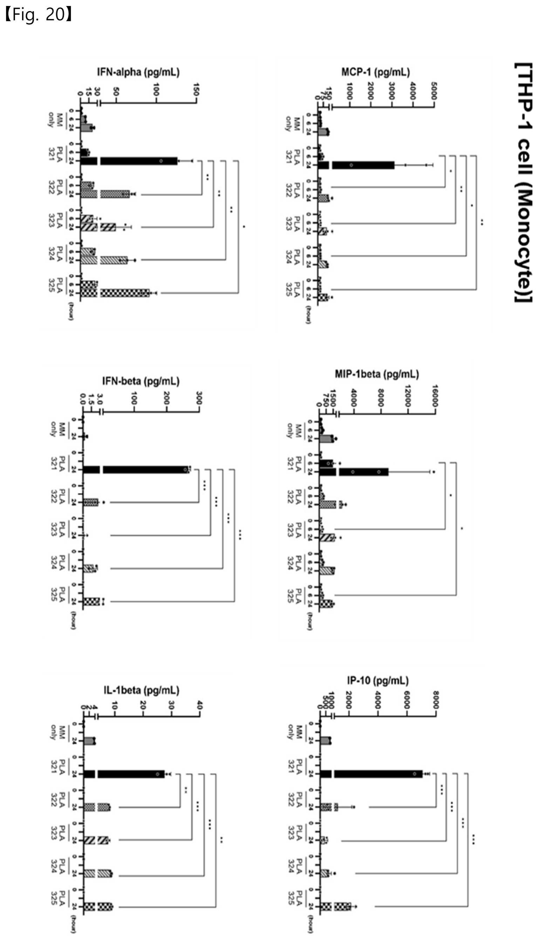

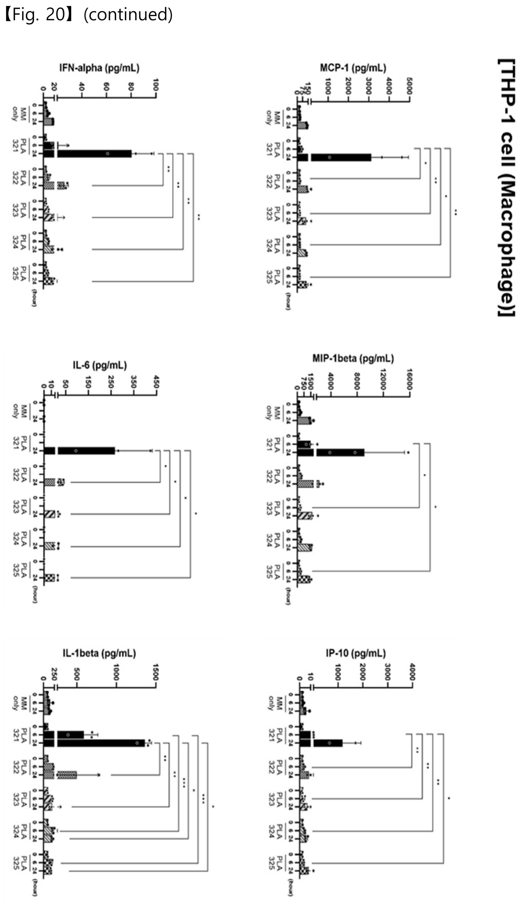

Compared to introduction of monocyte with an mRNA construct (PLA321) containing a continuous A79 in the form of a poly(A) tail, significantly lower levels of Type I interferon (IFN-α, IFN-β), inflammatory cytokines (IL-1β), and chemokines (MCP-1, MIP-1β, IP-10) were detected in the cell culture medium when the mRNA constructs according to the present invention (PLA322-325) were transduced into monocytes (FIG. 20).

Similarly, the mRNA construct according to the present invention having PMA-differentiated THP-1-derived macrophages introduced thereinto (PLA322-325) exhibited significantly lower levels of Type I interferon (IFN-α), inflammatory cytokines (IL-1β, IL-6), and chemokines (MCP-1, MIP-1β, IP-10) in the cell culture medium compared to the mRNA construct having an A₇₉-type poly(A) tail (PLA321) introduced thereinto (FIG. 20).

This suggests the potential for the application of the nucleic acid construct according to the present invention to induce a low-level innate immune response, which is a characteristic suitable for application in pharmaceutical compositions for vaccination or gene therapy.

### INDUSTRIAL APPLICABILITY

The nucleic acid construct platform according to the present invention exhibits enhanced stability and protein expression rates within cells without interference with CDS, UTR sequences, or the like, and thus is generally used in fields such as gene therapy and vaccination. Furthermore, it may be used to prevent poly A cleavage that may occur during production of mRNA formulations from the pDNA state, thereby producing intact mRNA formulations.

Although specific configurations of the present invention have been described in detail, those skilled in the art will appreciate that this detailed description is provided as preferred embodiments for illustrative purposes, and should not be construed as limiting the scope of the present invention. Therefore, the substantial scope of the present invention is defined by the accompanying filed claims and equivalents thereto.

### SEQUENCE LISTING FREE TEXT

An electronic file is attached.

## Claims

1. A nucleic acid construct comprising:
a coding region (CDS) encoding a polypeptide or protein; and
a poly(A) tail having any one selected from structures represented by Formula (I) to Formula (VIII):
{(A)ₘ-CS₁-(A)ₙ-CS₂-(A)ₒ}ₚ Formula (I)
{(N)_{q}-(A)ₘ-CS₁-(A)ₙ-CS₂}ₚ Formula (II)
[(A)ₘ-CS₁-{ (A) ᵣ-(G/C/U)}ₓ-(A)_{y}-CS₂]ₚ Formula (III)
[(A)ₘ-CS₁-{(A)ᵣ-(G/C/U)}ₓ-{(A)ₛ-(G/C/U)}_{x'}-=CS₂]ₚ Formula (IV)
[{ (A) ᵣ-(G/C/U)}ₓ-(A) _{y}-CS₁-(A) ₙ-CS₂]ₚ Formula (V)
[{(A)ᵣ-(G/C/U)}ₓ-(A) _{y}-CS₁-{(A) ᵣ-(G/C/U)}_{x'}-(A)_{y'}-CS₂]ₚ Formula (VI)
[{(A)ᵣ-(G/C/U)}ₓ-(A) _{y}-CS₁-{(A) ₛ-(G/C/U) }_{x'}-CS₂]ₚ Formula (VII)
[{(A) ᵣ-(G/C/U)}ₓ-(A) _{y}-CS₁-{(A) r- (G/C/U) }x'-{(A)ₛ-(G/C/U)}_{x'}-CS₂]ₚ Formula (VIII)
wherein G/C/U represents G (guanine), C (cytosine), or U (uracil),
wherein N represents U (uracil), G (guanine), A (adenine), or C (cytosine),
wherein m is an integer of 5 to 200, n is an integer of 3 to 200, o is an integer of 3 to 200, p is an integer of 1 to 10, q is an integer of 0 to 200, r and s are integers of 1 to 30, x and x' are integers of 1 to 10, y and y' are integers of 0 to 80, and
wherein CS₁ and CS₂ complementarily bind to each other.

2. The nucleic acid construct according to claim 1, wherein the poly A tail has the structure of Formula (I),
wherein m is an integer of 10 to 100, n is an integer of 10 to 50, o is an integer of 10 to 30, and p is an integer of 1 to 5.

3. The nucleic acid construct according to claim 1, wherein CS₁ and CS₂ complementarily bind to each other to form a stem-loop structure, a bulged stem structure, or a pseudoknot structure, or complementarily bind to each other by 30% to 100%.

4. The nucleic acid construct according to claim 3, wherein CS₁ is represented by any one of nucleotide sequences of SEQ ID NOs: 1 to 9.

5. The nucleic acid construct according to claim 3, wherein CS₂ is represented by any one of nucleotide sequences of SEQ ID NOs: 10 to 18.

6. The nucleic acid construct according to claim 1, wherein the poly A tail is represented by any one of nucleotide sequences of SEQ ID NO: 24 and SEQ ID NOs: 29 to 102.

7. The nucleic acid construct according to claim 1, comprising a modified nucleic acid.

8. The nucleic acid construct according to claim 7, wherein the modified nucleic acid is selected from the group consisting of pseudouridine (Ψ), N1-methylpseudouridine (m1Ψ), 5-methyluridine (m5U), 2-thiouridine (s2U), 2'-O-methyluridine (2'-O-methyl-U, Um), 5-methylcytidine (m5C), and 5-methoxyuridine (5moU).

9. The nucleic acid construct according to claim 1, further comprising:
a 5'-untranslated region (5'-UTR) and a 3'-untranslated region (3'-UTR) linked to both ends of the coding region; and
a 5'-cap or IRES linked to the 5'-untranslated region.

10. The nucleic acid construct according to claim 1, wherein the polypeptide or protein is:
(a) a therapeutically active protein or peptide; or
(b) an antigen selected from the group consisting of tumor antigens, pathogenic antigens, viral antigens, protozoal antigens, bacterial antigens, allergens, and autoimmune antigens.

11. A vaccine comprising the nucleic acid construct according to any one of claims 1 to 10.

12. A pharmaceutical composition for gene therapy comprising the nucleic acid construct according to any one of claims 1 to 10.
